# NEUE EUROPÄISCHE PATENTSCHRIFT

(11) **EP 2 788 171 B2**
(45) Veröffentlichungstag und Bekanntmachung des Hinweises auf die Entscheidung über den Einspruch: **17.06.2026**
(45) Hinweis auf die Patenterteilung: 17.02.2021
(21) Anmeldenummer: 12808303.7
(22) Anmeldetag: 10.12.2012
(51) Int. Cl.: B29C 67/20, A61L 27/56, C08J 9/26, C08J 9/28, A61L 27/20, A61L 27/22, A61L 27/38

(54) **HERSTELLUNG ANISOTROP STRUKTURIERTER WERKSTOFFE**
PRODUCTION OF MATERIALS HAVING AN ANISOTROPIC STRUCTURE
FABRICATION DE MATÉRIAUX STRUCTURÉS DE MANIÈRE ANISOTROPE

(30) Priorität: 08.12.2011 DE 102011120488
(43) Veröffentlichungstag der Anmeldung: 15.10.2014
(73) Patentinhaber: Stuckensen, Kai, 97082 Würzburg (DE)
(72) Erfinder: STUCKENSEN, Kai, 97082 Würzburg (DE); GBURECK, Uwe, 97638 Mellrichstadt (DE); GROLL, Jürgen, 97078 Würzburg (DE)
(86) Internationale Anmeldenummer: PCT/EP2012/074980
(87) Internationale Veröffentlichungsnummer: WO 2013/083844

(56) Entgegenhaltungen:
- EP-A1- 1 275 405
- WO-A1-2008/103017
- DE-A1- 19 751 031
- US-A1- 2003 209 486
- US-B1- 6 355 699

## Beschreibung

### Gebiet der Erfindung

Die Erfindung betrifft das Gebiet der Herstellung von Werkstoffen, insbesondere die Herstellung von medizinischen Werkstoffen.

### Hintergrund der Erfindung

Medizinische Werkstoffe werden beispielsweise für Implantate, Mikrosensoren und andere Produkte, die im menschlichen oder tierischen Körper platziert werden, verwendet. Dabei treten die medizinischen Werkstoffe unmittelbar mit dem Gewebe und den Zellen des Körpers in Kontakt. In ihrer natürlichen Umgebung sind Zellen von einer extrazellulären Matrix umgeben, welche für das Überleben der Zellen wichtig ist, da sie deren Adhäsion, Proliferation, Migration, Differenzierung und Funktion maßgeblich beeinflusst. Hauptkomponenten der extrazellulären Matrix sind Hydrogele und nicht wasserlösliche Polymerfasern, welche als mechanisches Gerüst dienen. Hinzu kommen Basalmembranen und ultradünne Trennschichten zwischen Geweben. Diese Strukturen sind auf die unterschiedlichen biologischen Anforderungen verschiedener Organe und Gewebe abgestimmt. Um eine gute Interaktion zwischen den Zellen und den medizinischen Werkstoffen zu fördern, wird versucht, die Werkstoffe der natürlichen Umgebung der Zellen in Struktur und Zusammensetzung anzupassen.

Die DE 19751031 A1 beschreibt hochreine Kollagenschwammprodukte mit offener Porenstruktur, die ein Einwachsen der Zellen in den Schwamm ermöglichen sollen. Zur Herstellung der Kollagenschwämme wird ein Einfrierverfahren verwendet mit dem eine homogene und gezielte Verteilung der Kollagenfasern in kanalähnlichen Leitstrukturen erzeugt wird, indem fingerförmige Eiskristalle durch eine Kollagen-Typ I Dispersion wachsen. Hierzu wurden, analog zu gängigen Verfahren der Metallverarbeitung, Gefrierverfahren konzipiert, welche eine Mischung von Substanzen zwischen zwei temperierbaren, parallel, bzw. konzentrisch zueinander angeordneten Flächen strukturieren, indem der Temperaturgradient zwischen den Flächen konstant gehalten wurde. Die auf diese Weise strukturierte Mischung wird anschließend gefriergetrocknet, indem sie unter Überdruck vorgekühlt und schlagartig entspannt wird. Die so gewonnenen Werkstoffe bestehen allerdings ausschließlich aus einer einzigen funktionellen Komponente, nämlich Kollagen Typ I und können daher die natürliche Umgebung von Zellen nur bedingt nachbilden.

Tampieri et al., 2008 beschreiben die Verbindung von drei Lagen unterschiedlich zusammengesetzter Kollagenscaffolds zu einer Gesamtstruktur, wobei jeder Scaffold eine andere Knochen- bzw. Knorpelschicht repräsentiert. Die oberste Lage besteht aus reinem Kollagen I und dient als Chondralzonen-Ersatz. Den subchondralen Knochen ersetzt, durch Fällungsreaktion mit Hydroxylapatit mineralisiertes Kollagen I, mit einem Mineral-Matrix-Verhältnis von 70/30 Gew.-%. Eine auf gleiche Weise, im Mineral-Matrix-Verhältnis von 40/60 Gew.-%, mineralisierte Zwischenschicht unterscheidet sich jedoch deutlich von der Tidemark des nativen Knorpelgewebes. Das Hydroxylapatit der Subchondralzone ist dabei teilweise durch Magnesium dotiert. Die einzelnen Kollagenscaffolds wurden jeweils separat durch 1,4-Butandioldiglycidylether (BDDGE) vernetzt und unter Verwendung eines "Webeprozesses" miteinander verbunden. Anschließend wurde die Gesamtstruktur gefriergetrocknet. Zellversuche zeigten aber, dass die so hergestellte Struktur nur eine begrenzte Besiedelung durch Zellen erlaubt. So konnte lediglich eine geringe Zellmigration in das Innere der Knorpelersatzzone mit einer uneinheitlichen Verteilung beobachtet werden, wohingegen die Region im Zentrum des Scaffolds im Wesentlichen azellulär blieb. Eine vollständige Integration des Werkstoffs nach der Implantation ist daher nicht möglich.

Die EP 1858562 B1 beschreibt einen porösen, dreischichtigen, osteochondralen Scaffold. Dieser umfasst eine mit einer glatten Oberfläche überzogene Zone, die zu 100 % aus Kollagen I equinen Ursprungs besteht und dem Knorpelgewebe entsprechen soll. Der Bereich der Subchondral- und Knochenzone wird durch Komposite aus Kollagen Typ I und nanostrukturierten, magnesiumangereicherten Hydroxylapatiten dargestellt. Die einzeln hergestellten Zonen werden jedoch lediglich nachträglich durch einen Kompressionsvorgang zusammengefügt, wodurch es bei einer Rehydratation der gefriergetrockneten Matrizes zu einer Delamination kommen kann. Aus histologischen Ergebnissen von Tierversuche geht zudem hervor, dass in der Knorpelzone nur fibröses Knorpelgewebe, jedoch kein natives Gelenkknorpelgewebe gebildet wird. Solch fibröses Knorpelgewebe entsteht vor allem bei den natürlichen aber seltenen und unvollständigen Selbstheilungsprozessen des Knorpels. Es enthält neben dem hauptsächlich im gesunden Gelencknorpel vorkommenden Kollagen Typ II auch untypisches Kollagen Typ I und unterscheidet sich in Struktur und Funktionalität maßgeblich von nativem Knorpelgewebe. Die nachteilige Bildung von fibrösem Knorpelgewebe ist möglicher Weise darauf zurückzuführen, dass die Knorpelersatzzone nur aus einer einzelnen Schicht besteht, welche im Vergleich zu nativem Knorpel eine untypische Zusammensetzung, sowie zu große Porositäten mit einer unnatürlichen Ausrichtung besitzt.

DE 197 51 031 A1 offenbart ein Verfahren zur Herstellung eines Werkstoffs mit anisotropen Poren und WO 2008/103017 A1 offenbart einen Werkstoff .

Es besteht daher der Bedarf nach stabilen Werkstoffen mit funktionell unterschiedlichen Bereichen, welche die natürliche Umgebung von Zellen nachbilden.

### Zusammenfassung der Erfindung

Die Erfindung betrifft ein Verfahren zur Herstellung eines mehrschichtigen Werkstoffs gemäß Anspruch 1.

### Beschreibung der Figuren

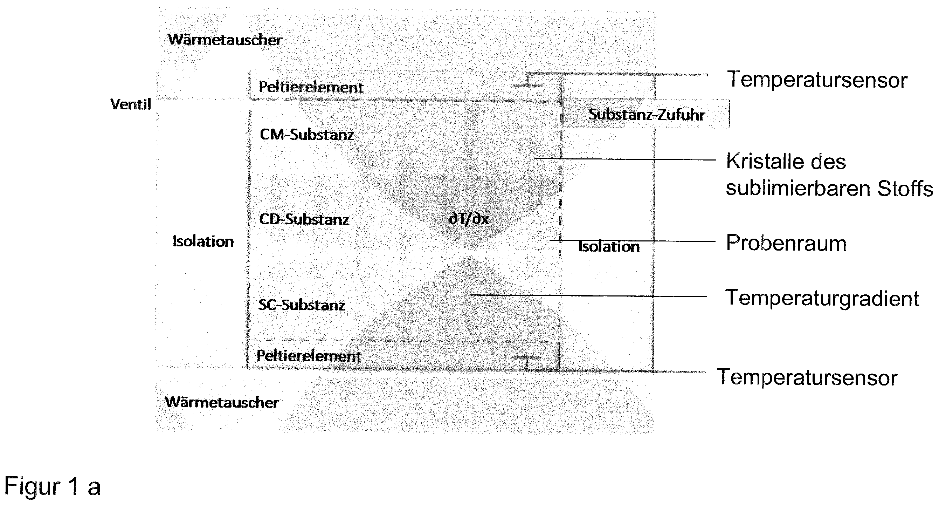

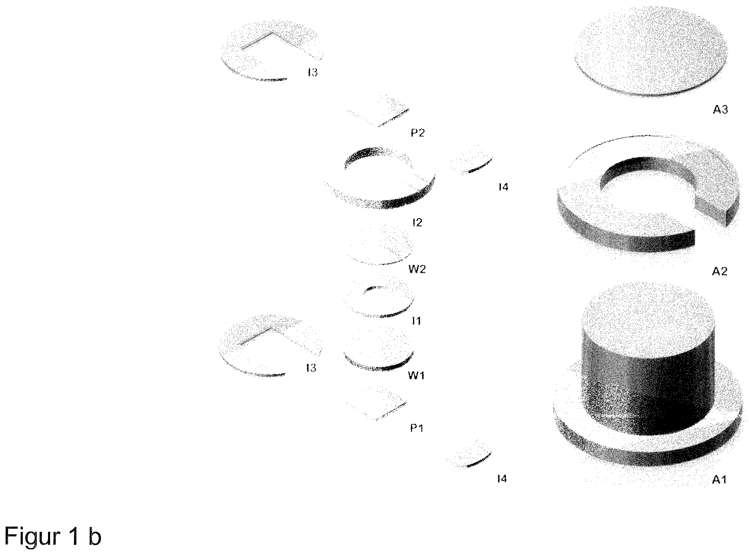

Figur 1 zeigt den Aufbau und die grundlegende Funktionsweise einer möglichen Ausführung einer Erstarrungsapparatur, a) Vereinfachte schematische Darstellung der Funktionsweise. An Wärmetauscher gekoppelte Peltierelemente sorgen über einen angesteuerten äußeren Temperaturgradienten für ein anisotropes Kristallwachstum in den Substanzen im Probenraum. b) Bauplan einer möglichen Ausführung einer Erstarrungsapparatur. Der Probenraum befindet sich in Zentrum der Isolationseinheit I1 und steht in thermischem Kontakt mit den temperierbaren Körpern W1 und W2, die ober- und unterhalb des Probenraums angebracht sind. Die temperierbaren Körper befinden sich innerhalb der Isolationseinheit I2 und sind an die Peltierelemente P1 und P2 gekoppelt, welche durch die Isolationseinheiten I3 und I4 fixiert werden. Diese innere Baugruppe befindet sich im Zentrum des Wärmetauscherrings A2, welcher zusammen mit den Wärmetauschereinheiten A1 und A3 eine äußere Baugruppe bilden.

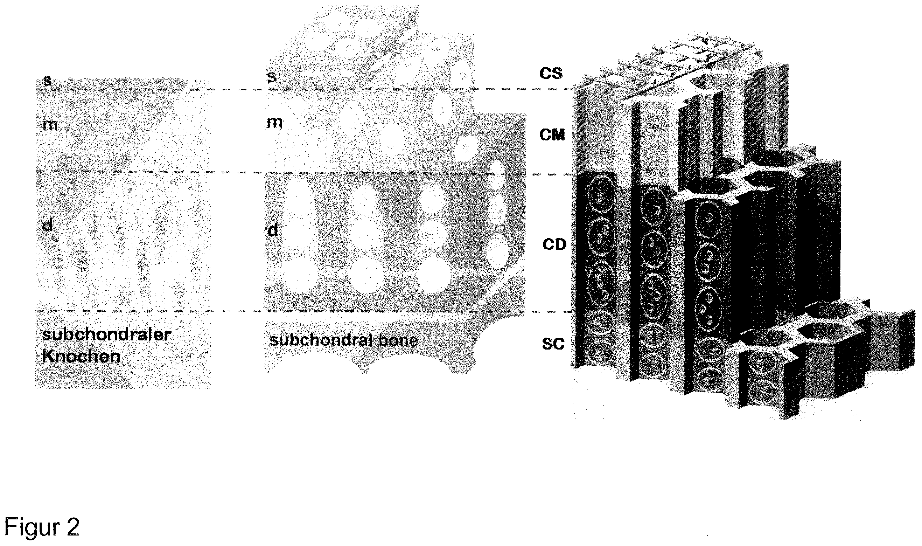

Figur 2 zeigt eine histologische (links) und eine schematische (mitte) Darstellung der nativen Gelenkknorpelstruktur mit drei unterscheidbaren Zonen (s: superficial (oberflächlich); m: middle (mittig); d: deep (tief)) (Klein et al., 2009), sowie eine in Schichten aufgebaute biomimetische Trägermatrix mit durchgängigen Poren (rechts). Vertikal bogenartig verlaufende gestrichelte Linien in der schematischen Darstellung der Gelenkknorpelstruktur symbolisieren die Orientierung von Kollagenfasern. Die Chondrozyten sind in ihrer Gestalt (Sphäroide) und Organisation den spezifischen Anforderungen der jeweiligen Zone (s, m und d) unterschiedlich angepasst. In der Trägermatrix ist der Knochenersatzkomposit (SC) mit einer unteren Chondralzone (CD) verbunden, welche in eine mittlere Chondralzone (CM) übergeht. Diese ist wiederum mit funktionalisierten Polymerfasern verbunden, welche eine abschließende Gleitschicht (CS) ausbilden.

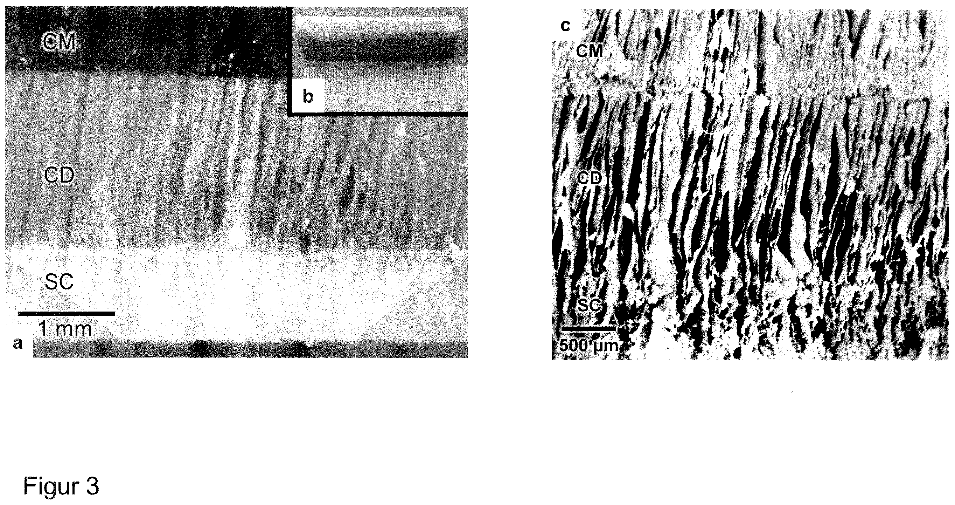

Figur 3 zeigt lichtmikroskopische (a), eine photographische (b) sowie eine elektronenmikroskopische Aufnahme (c) einer monolithischen, osteochondralen Trägerstruktur auf Alginatbasis. Anisotrope Poren, mit einem Querschnitt im Größenbereich von 80 µm, verlaufen kontinuierlich durch die einzelnen Zonen des Chondralteils (CM - obere, dunkel dargestellte Zone und CD - mittlere, grau dargestellte Zone) sowie durch den Subchondralteil (SC - untere, weiß dargestellte Zone). Der Subchondralteil ist durch die resorbierbare Calciumphosphatphase Bruschit mineralisiert. Einzelne Zonen wurden mit eingefärbten Vorstufen hergestellt.

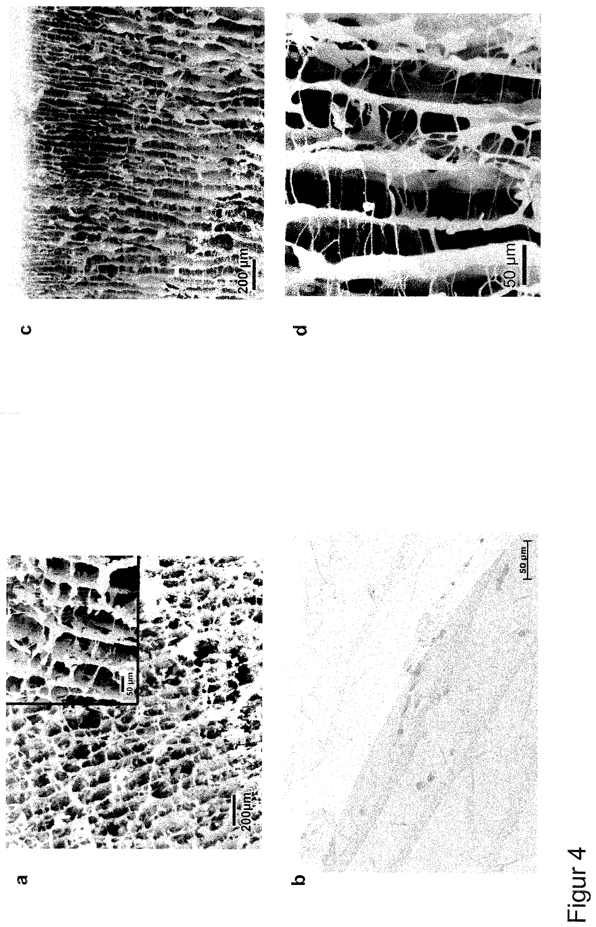

Figur 4 a zeigt eine elektronenmikroskopische Aufnahme der Oberfläche einer chondralen Trägermatrix, die gemäß Beispiel 2 hergestellt wurde. Figur 4 b zeigt einen Hämatoxylin-Eosin-gefärbten histologischen Schnitt durch das Zentrum der Chondralzone einer 21 Tage durch humane mesenchymale Stammzellen besiedelten Alginatmatrix. Nach statischer Zellbesiedelung sind die Zellen in das Innere der Trägermatrix migriert und dort bevorzugt an Mikrostrukturen innerhalb der Poren adhäriert. Syntheseprodukte in Form von zellgebildeter Matrix sind bereits zu erkennen. Figur 4 c zeigt eine lichtmikroskopische Aufnahme einer Aufsicht auf eine Kollagen-basierte chondrale Trägerstruktur. Die Trägermatrix besteht überwiegend aus Kollagen und Chondroitinsulfat und ist von anisotropen, langgestreckten Poren durchzogen. Figur 4 d zeigt eine rasterelektronenmikroskopische Aufnahme eines Querschnittes durch die Trägermatrix von Figur 4 c.

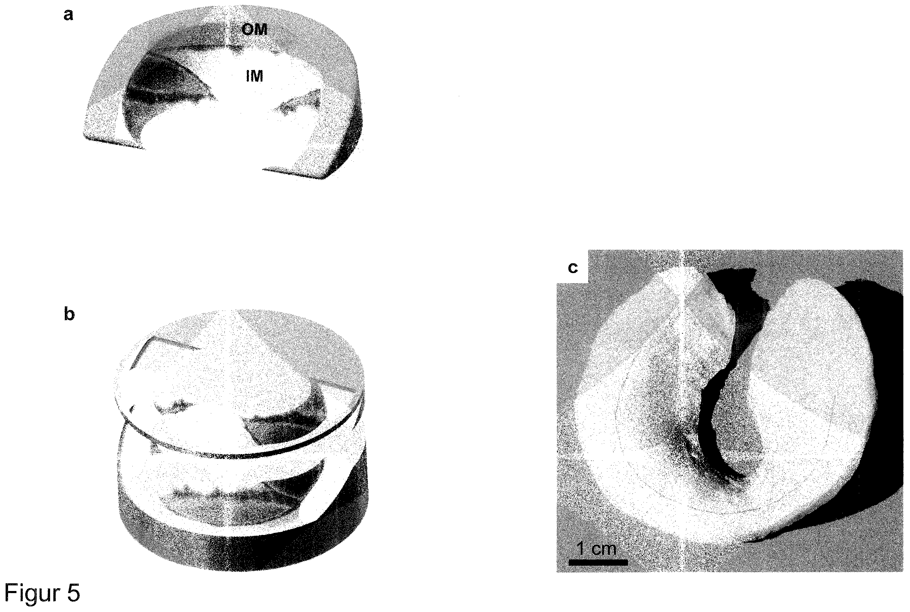

Figur 5 zeigt die schematische Darstellung einer Trägermatrix zur Behandlung von Meniskusdefekten (a), die Geometrie eines temperierbaren Körpers (unten) und eines isolierenden Körpers (oben) zur Herstellung einer solchen Trägermatrix (b) und ein Alginatmodell eines lateralen Meniskus (c). Die eingezeichnete Linie in Teilbild c verdeutlicht den Verlauf der Porenstruktur. Die Trägermatrix besteht aus einem äußeren Meniskusbereich (OM) sowie einem inneren Meniskusbereich (IM), welche sich in ihren chemischen Zusammensetzungen unterscheiden.

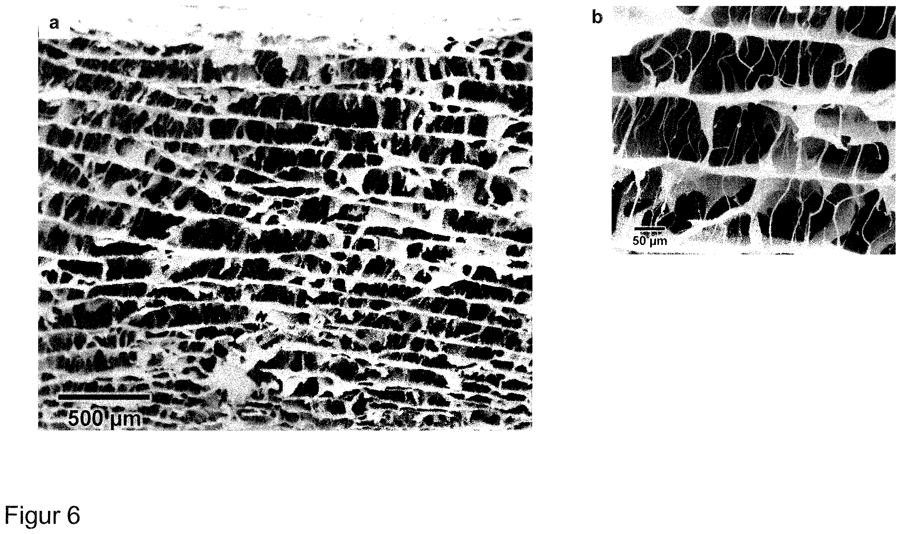

Figur 6 zeigt rasterelektronenmikroskopische Aufnahmen eines Vertikalschnitts durch eine Kollagen-Trägermatrix zur Behandlung von Meniskusdefekten (Beispiel 4).

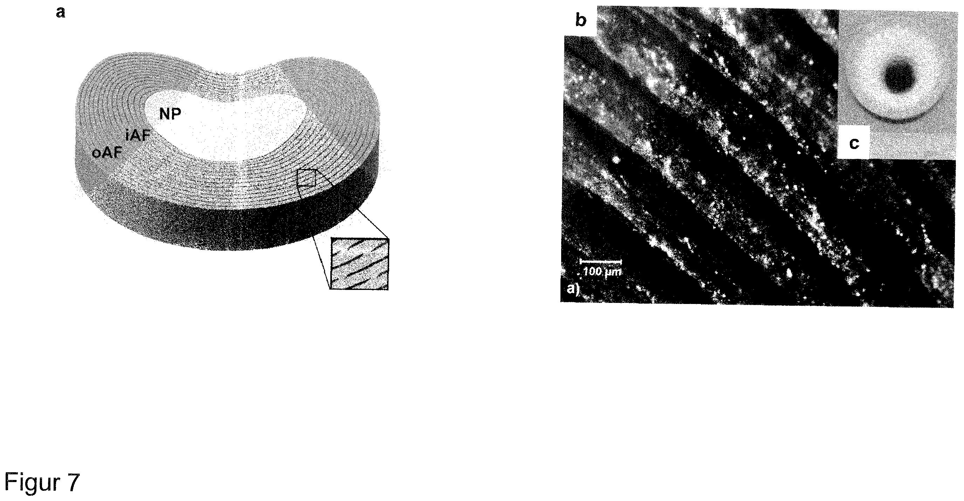

Figur 7 a zeigt die schematische Darstellung einer Trägermatrix zur Behandlung von Bandscheibendefekten. Entsprechend dem nativen Gewebe weist die Trägermatrix Bestandteile der extrazellulären Knorpelmatrix auf, welche zu einer biomimetischen, monolithischen Matrix vereinigt sind. Ein ungerichtetes Netzwerk, das dem Nucleus pulposus (NP) entspricht, befindet sich innerhalb eines durch gerichtete Erstarrung hergestellten Faserarrangements mit lamellarem Aufbau, welches dem Anulus fibrosus entspricht. Dieses ist wiederum in einen äußeren Anulus fibrosus (oAF) und in einen inneren Anulus fibrosus (iAF) untergliedert. Die Detailansicht zeigt den prinzipiellen Aufbau der Mikrostruktur des Anulus fibrosus, welche aus einzelnen Lamellen besteht, die untereinander verbunden sind. Figur 7 b und c zeigen eine monolithische, bandscheibenartige Trägerstruktur auf Alginatbasis, wobei anisotrope Lamellen die Struktur des äußeren Bereichs der Trägermatrix prägen.

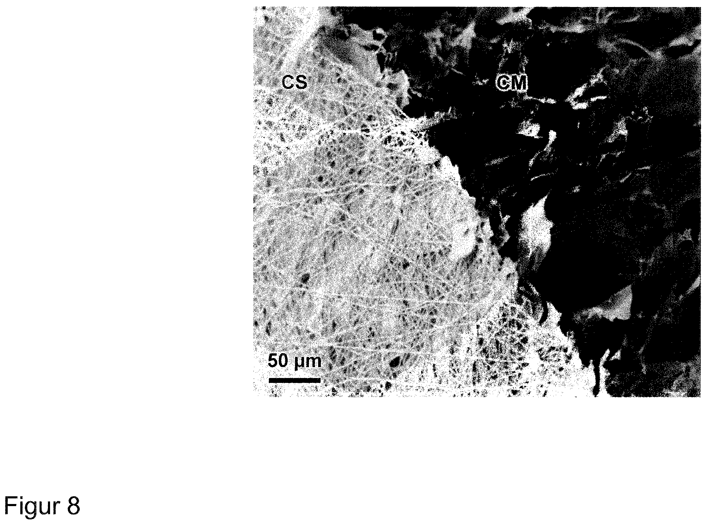

Figur 8 zeigt ein rasterelektronenmikroskopisches Schnittbild von funktionalisierten Polymerfasern welche die abschließende Gleitschicht (CS) einer darunterliegenden osteochondralen Trägermatix bilden und an die mittlere chondrale Zone (CM) gebunden sind. Die Polymerfasern verlaufen orthogonal zu der Porenstruktur der restlichen Trägermatrix und bilden so die Faseranordnung des nativen Osteochondralgewebes nach.

### Detaillierte Beschreibung der Erfindung

In einem ersten Aspekt betrifft die Erfindung ein Verfahren zur Herstellung eines mehrschichtigen Werkstoffs mit anisotropen Poren, umfassend die Schritte Bereitstellen eines Temperaturgradienten durch zwei einander gegenüber liegend angeordnete temperierbare Körper; Anordnen und Erstarren einer ersten Substanz, die mindestens einen sublimierbaren Stoff enthält, in dem Temperaturgradienten, um eine erste Schicht zu bilden; Anordnen und Erstarren mindestens einer weiteren Substanz, die mindestens einen sublimierbaren Stoff enthält, in dem Temperaturgradienten, um mindestens eine weitere Schicht zu bilden, die an die vorhergehende Schicht anschließt; Sublimieren des Stoffs und Verfestigen der Schichten.

Das Verfahren dient der Herstellung eines Werkstoffs, der mehrere, das heißt mindestens zwei, bevorzugt drei, unterschiedliche Schichten umfasst, die zu einem Monolithen vereinigt werden. Die Schichten können sich sowohl in ihrer Zusammensetzung, ihrer Funktionalität als auch ihren physikalischen Eigenschaften unterscheiden. Sie gleichen insofern biologischen Geweben, wie beispielsweise extrazellulären Matrices, die ebenfalls Bereiche bzw. Schichten unterschiedlicher chemischer Zusammensetzung und biologischer Funktionalität aufweisen. Dementsprechend sind die mit dem Verfahren hergestellten Werkstoffe insbesondere für medizinische Anwendungen, auch innerhalb des tierischen oder menschlichen Körpers, geeignet. Durch die unterschiedlichen Schichten können sie die natürliche Umgebung, in die sie eingebracht werden, nachbilden und gegebenenfalls entsprechende biologische Funktionen erfüllen.

Die unterschiedlichen Schichten werden gebildet, indem mehrere Substanzen über- oder nebeneinander angeordnet und erstarrt werden. Um eine durchgängige anisotrope Porenstruktur zu erhalten, werden die Substanzen auf- oder nebeneinander angeordnet, ohne dass der Erstarrungsprozess unterbrochen wird.

Die Substanzen werden erstarrt, indem sie in einen Temperaturgradienten angeordnet werden, der durch zwei einander gegenüber liegend angeordnete temperierbare Körper gebildet wird. Unter einem Erstarrungsprozess ist hierbei zu verstehen, dass die Freiheitsgrade für eine räumliche Bewegung von Teilchen derart eingeschränkt werden, dass sich diese, wenn überhaupt, nur noch in sehr geringem Maße räumlich bewegen können. Dies geht in vielen Fällen mit einer Phasenumwandlung einher. Die Erstarrung kann gerichtet oder ungerichtet erfolgen, wobei bei einer ungerichteten Erstarrung eine makroskopisch isotrope Struktur und bei einer gerichteten Erstarrung eine makroskopisch anisotrope Struktur entsteht. Indem die Substanzen in einem direktionalen Temperaturgradienten erstarrt werden, kommt es zu einer gerichteten Erstarrung, wobei die Erstarrung an den Stellen der Substanzen mit der tiefsten Temperatur beginnt, und sich mit der Zeit in Richtung der Stellen der Substanzen mit der höchsten Temperatur hin bewegt. Dabei wandert die Erstarrungsfront gleichmäßig durch die Substanzen. Das Verfahren kann beispielsweise mit einer Erstarrungsapparatur wie in Figur 1 gezeigt durchgeführt werden.

Während des Erstarrens bildet der sublimierbare Stoff in den Substanzen Kristalle aus, die ebenfalls gerichtet entlang des Temperaturgradienten wachsen können. Indem die Schichten nacheinander angeordnet werden, wachsen die Kristalle des sublimierbaren Stoffs sukzessive durch alle Schichten gleichmäßig hindurch (Figur 1 a). Durch die Sublimation werden die Kristalle des sublimierbaren Stoffs aus den gebildeten Schichten entfernt, so dass hohle Poren zurückbleiben. Aufgrund der gerichteten Erstarrung haben die Poren eine anisotrope Struktur und verlaufen kontinuierlich durch alle Schichten hindurch. Eine durchgängige anisotrope Porenstruktur ermöglicht die Besiedelung des Werkstoffs durch Zellen bis ins Innere, wodurch eine gute Integration und Funktionalität des Werkstoffs im nativen Gewebe ermöglicht wird.

Die erstarrten Schichten, sowie aufgebrachte Polymerfaserschichten, können durch geeignete Verfahren verfestigt werden, indem zusätzliche Verknüpfungen zwischen den Polymeren bzw. zwischen den von ihnen gebildeten Strukturen gebildet werden. Durch diese zusätzlichen Verbindungen entsteht ein dreidimensionales Netzwerk, welches die Härte und Stabilität des Werkstoffs erhöht. Das Verfestigen kann durch verschiedene chemische Verfahren erfolgen, beispielsweise durch nasschemische Quervernetzung, Dehydrothermalverfahren, enzymatische Quervernetzung, nicht enzymatische Glykation, UV-Bestrahlung, Gammabestrahlung, Sinterung, Infiltration des Werkstoffs oder durch eine Kombination verschiedener Verfahren. Die nasschemische Quervernetzung erfolgt bevorzugt mittels Carbondiimiden, Isocyanaten, komplexbildenden Ionen oder Glutaraldehyd, ferner bevorzugt bei einem Druck von ≤ 300 mbar. In einer bevorzugten Ausführungsform umfasst das Verfestigen der Schichten eine nasschemische Quervernetzung und ein Dehydrothermalverfahren.

In einer bevorzugten Ausführungsform beträgt der Temperaturgradient zwischen 0,5 K/mm und 200 K/mm, bevorzugt zwischen 2,5 K/mm und 25 K/mm, ferner bevorzugt zwischen 5 K/mm und 15 K/mm. Solche Gradienten erlauben ein kontinuierliches gerichtetes Kristallwachstum, welches zur Bildung anisotroper Poren führt. Der Temperaturgradient wird bestimmt durch die Temperaturen der beiden temperierbaren Körper und deren Abstand zueinander, wobei ein temperierbarer Körper die tiefste Temperatur und der gegenüberliegende die höchste Temperatur besitzt und somit den Gradienten erzeugt. Der Temperaturgradient bestimmt maßgeblich die Geschwindigkeit, mit der die Substanzen erstarren und beeinflusst damit auch die Bildung der Kristalle des sublimierbaren Stoffs. Da die Kristalle des sublimierbaren Stoffs unmittelbar Form und Größe der Poren bestimmen, kann die Gestalt der Poren durch den Temperaturgradienten beeinflusst werden. So führen beispielsweise stärkere Temperaturgradienten zur Bildung kleinerer und schmälerer Poren bei Erstarren derselben Substanz. Daher werden stärkere Gradienten tendenziell bei tieferen Temperaturen eingesetzt. Bei tieferen Temperaturen können zudem stärkere Temperaturgradienten entstehen wenn das wärmere Temperaturniveau wegen der Möglichkeit von Proteindenaturierungen nach oben beschränkt ist. Ein schwacher Temperaturgradient, kann zu einer langsamen Gefriergeschwindigkeit führen, welche die Bildung von Kristallen mit kolumnarer Morphologie fördert. Eine schnelle Gefriergeschwindigkeit kann hingegen zur Bildung dendritischer Kristalle mit vielen Verzweigungen führen. Dies kann genützt werden, um eine natürliche Gewebestruktur, die sowohl isotrop als auch anisotrop strukturierte Bereiche umfasst, wie beispielsweise eine Bandscheibe, nachzubilden. Eine isotrope Porenstruktur kann durch equiaxiale-dendritisches Kristallwachstum erzeugt werden, während eine anisotrope Porenstruktur durch kolumnares oder dendritisches Kristallwachstum erzeugt werden kann. Werkstoffe mit anisotropen Poren haben, bei den besonderen Belastungen wie sie in spezialisierten Geweben auftreten, eine höhere Stabilität gegenüber solchen mit ungerichteter, isotroper Porenstruktur und sind bei gleicher Porosität druck- und zugfester. Zudem erlauben anisotrope Poren, im Gegensatz zu isotropen Poren, eine effektive Zellmigration ins Innere des Werkstoffs. Soll der Werkstoff dennoch isotrope Poren aufweisen, beispielsweise um eine natürliche isotrope Struktur nachzubilden, kann dies durch eine ungerichtete Erstarrung der Substanzen erreicht werden. Dazu werden die Substanzen bei einer einheitlichen Temperatur oder einem sehr geringen Temperaturgradienten von < 0,5 K/mm erstarrt. Es ist auch möglich Bereiche bzw. Schichten mit isotroper Struktur und solche mit anisotroper Struktur innerhalb desselben Werkstoffs auszubilden, indem einzelne Bereiche des Werkstoffs gerichtet bzw. ungerichtet erstarrt werden.

In einer bevorzugten Ausführungsform werden die Substanzen mit einer linear interpolierten Abkühlrate von 2 K/min bis 45 K/min, bevorzugter 5 K/min bis 35 K/min erstarrt. Unter der Abkühlrate ist die lineare Interpolation der Temperaturdifferenz pro Zeit der zu erstarrenden Substanzen, vom Einsetzen des Kristallwachstums bis zur vollständigen Erstarrung, zu verstehen.

In einer vorteilhaften Ausführungsform ist der Temperaturgradient während des Verfahrens konstant. Dies bedeutet dass die Temperaturdifferenz zwischen der tiefsten Temperatur, das heißt dem einen temperierbaren Körper, und der höchsten Temperatur, das heißt dem gegenüberliegend angeordneten temperierbaren Körper, sowie der räumliche Abstand zwischen beiden unverändert bleibt. Dadurch wird ein stabiles Kristallwachstum durch alle Schichten hindurch gewährleistet. Ein konstanter Temperaturgradient schließt auch ein, dass die tiefste und die höchste Temperatur, bei gleichbleibendem Abstand der beiden temperierbaren Körper, parallel erhöht oder erniedrigt werden.

In einer vorteilhaften Ausführungsform des Verfahrens wird der Temperaturgradient, bei gleichbleibendem Abstand der temperierbaren Körper, durch Erhöhung oder Erniedrigung der Temperatur der temperierbaren Körper, während des Verfahrens vergrößert oder verkleinert. Vorzugsweise erfolgt dies nach der Erstarrung einer oder mehrerer Schichten und vor der Zuführung einer weiteren Substanz.

In einer bevorzugten Ausführungsform liegt die tiefste Temperatur des Temperaturgradienten zwischen -200°C und +90°C und die höchste Temperatur des Temperaturgradienten zwischen +100C und -25°C. In einer ferner bevorzugten Ausführungsform liegt die tiefste Temperatur des Temperaturgradienten zwischen - 60°C und -15°C und die höchste Temperatur des Temperaturgradienten zwischen +30°C und +5°C. Die tiefste und die höchste Temperatur des Gradienten richten sich nach der Zusammensetzung der Substanzen und dem Schmelzpunkt des sublimierbaren Stoffs. Insbesondere Zusammensetzungen mit hoher Dichte, beispielsweise Zusammensetzungen mit einem hohen Anteil an Polymeren, erstarren langsam und werden daher unter Temperaturgradienten erstarrt, welche bei tieferen Temperaturen erzeugt werden. Beinhalten die Substanzen einen sublimierbaren Stoff mit tiefem Schmelzpunkt, beispielsweise einem organischen Lösungsmittel, werden die Substanzen bevorzugt bei entsprechend tiefen Temperaturen erstarrt. Werden hingegen sublimierbare Stoffe wie beispielsweise Wasser oder Essigsäure verwendet, sind höhere Temperaturen möglich. Aufgrund der konstitutiven Unterkühlung an der Erstarrungsgrenze ist auch eine Kristallbildung von Wasser bei über 0°C möglich.

In einer bevorzugten Ausführungsform sind die tiefste und die höchste Temperatur des Temperaturgradienten während des Verfahrens konstant. Der Temperaturgradient, das heißt die Temperatur der beiden temperierbaren Körper und ihr Abstand zueinander wird vor dem Anordnen der ersten Substanz festgelegt. Während des Erstarrens der Substanzen bleiben sowohl die tiefste als auch die höchste Temperatur des Temperaturgradienten, das heißt die Temperaturen der temperierbaren Körper, und der Abstand der Körper zueinander unverändert. Eine Absenkung der Temperaturen beider temperierbaren Körper bei gleichbleibender Temperaturdifferenz während des Gefriervorgangs ist möglich aber nicht notwendig.

Der Begriff "temperierbare Körper" bezeichnet sowohl Körper, die aktiv Wärme abführen können, wie beispielsweise Peltierelemente, als auch solche Körper, die mittelbar Wärme abführen indem sie gekühlt, oder zuführen indem sie geheizt werden. Die temperierbaren Körper bestehen vorzugsweise aus Metall oder Metallverbindungen. Die Körper können sowohl horizontal als auch vertikal angeordnet sein, wobei bei einer horizontalen Anordnung regelmäßig der untere temperierbare Körper die tiefste Temperatur des Temperaturgradienten bestimmt. Die temperierbaren Körper können in individuellen Geometrien vorliegen, beispielsweise als Negativform für den herzustellenden Werkstoff. Neben herkömmlichen Formen wie Quader, Zylinder, Pyramiden, Kegel, Rotationsellipsoide, Kugeln, Ringe, oder deren Teilmengen in massiver Form oder Hohlkörperform sind auch individualisierte Formen möglich. Letztere können beispielsweise nach Informationen dreidimensionaler bildgebender Verfahren wie Röntgentomographie oder Magnetresonanztomographie angefertigt werden, so dass für jeden Patienten individuell angepasste Werkstoffe hergestellt werden können. Neben temperierbaren Körpern können zur Formgebung des Werkstoffs auch isolierende Körper verwendet werden. Diese sind vor allem geeignet einzelnen Schichten oder dem gesamten Werkstoff eine individuelle Form zu geben. Die temperierbaren bzw. isolierenden Körper können in direktem thermischen Kontakt mit den Substanzen stehen.

In einer bevorzugten Ausführungsform werden die Substanzen in einem Behältnis angeordnet, welches in dem Temperaturgradienten platziert wird. Der Werkstoff wird in diesem Fall innerhalb des Behältnisses gebildet und seine Form durch dessen Geometrie bestimmt. Wie die temperierbaren und isolierenden Körper kann auch das Behältnis als Negativform für den Werkstoff dienen und dementsprechend die oben genannten Formen aufweisen.

In einer bevorzugten Ausführungsform ist die erste und/oder weitere Substanz unabhängig voneinander eine Lösung, eine Dispersion, eine Suspension, ein Gel, eine Polymerschmelze, oder eine Mischung davon. Der Begriff "Substanz" bezeichnet die fließfähigen Vorstufen der Schichten des späteren Werkstoffs.

In einer bevorzugten Ausführungsform enthält die erste und/oder eine weitere Substanz unabhängig voneinander mindestens ein Polymer oder dessen Monomere. Die Zusammensetzung der Vorstufen bestimmt die Zusammensetzung der einzelnen Schichten des Werkstoffs. Die Inhaltsstoffe der Substanzen werden daher entsprechend der Funktion und den Eigenschaften, welche die einzelnen Schichten im späteren Werkstoff aufweisen sollen, gewählt. Bevorzugt entsprechen die Schichten dem Gewebe, welches der Werkstoff ersetzt oder in welches er integriert wird. Polymere sind bevorzugte Bestandteile der Substanzen, da sie durch intermolekulare Verbindungen stabile Strukturen und Netzwerke ausbilden. Vorzugsweise ist das Polymer ein natives Polymer. Im Gegensatz zu denaturierten Polymeren liegen native Polymere in ihrer natürlichen Sekundärstruktur vor, welche dem Molekül die effektive Bildung von Komplexen und Netzwerken ermöglicht und von Zellen als native Umgebung erkannt werden können. Ferner können Präpolymere und Makromonomere verwendet werden, wobei das Präpolymer ein künstliches (Co-)polymer mit einem Molekulargewicht von weniger als 50 kDa darstellt, das daneben vernetzungsfähige Gruppen wie beispielsweise (Meth)acrylate, Thiole, Isocyanate, Azide, Ethine, Aldehyde, Carbonsäuren und/oder Amine, enthält.

In einer bevorzugten Ausführungsform ist das Polymer ausgewählt aus der Gruppe bestehend aus Peptiden, Proteinen, bevorzugt Strukturproteinen, und Polysacchariden. Das Polymer kann ein synthetisch hergestelltes Polymer sein. Die Verwendung proteinogener Polymere, insbesondere die Verwendung von Strukturproteinen, ist vor allem für die medizinische Anwendung bevorzugt, da diese Polymere auch in der natürlichen Gewebematrix vorhanden sind. Durch die Verwendung solcher Polymere zur Herstellung des Werkstoffs ist es möglich die körpereigenen Strukturen, auch hinsichtlich ihrer chemischen Zusammensetzung, nachzubilden. Zudem bilden proteinogene Polymere, insbesondere Strukturproteine, stabile Netzwerke, welche für die Herstellung von Werkstoffen besonders geeignet sind. Kollagene bilden Triplehelices, welche sich zu langen Fasern zusammenlagern, wohingegen Keratine Superhelices formen, die wiederum intermediäre Filamente bilden. Neben proteinogenen Polymeren sind auch Polysaccharide zur Bildung stabiler Werkstoffe geeignet, da sie ebenfalls intermolekulare Netzwerke, wie Mikrofibrillen, formen. Die Bildung derartiger Strukturen trägt maßgeblich zur Stabilität des Werkstoffs bei. Neben strukturbildenden Polymeren können die Substanzen auch weitere Bestandteile, beispielsweise der extrazellulären Matrix, beinhalten. Besonders bevorzugt ist die Verwendung von Glycoaminoglycanen, wie beispielsweise Hyaluronsäure und Chondroitinsulfat, welche durch ihre starke Hydratisierung große Mengen an Wasser speichern können, die ein Vielfaches ihres Eigenvolumens übersteigen. Da die natürliche Gewebematrix sehr wasserhaltig ist, ist es vorteilhaft solche Stoffe in den Werkstoff einzubinden. Durch die Verwendung derartiger Polymere kann zudem eine, bei Deformation in wässrigem Milieu verstärkt auftretende, elektrostatische Abstoßung ausgenutzt werden, welche eine stoßdämpfende Wirkung der Trägermatrix erzeugen kann. Dadurch werden die physikalischen Eigenschaften des Werkstoffs der natürlichen Gewebematrix angeglichen.

In einer bevorzugten Ausführungsform ist das Polymer ausgewählt aus der Gruppe bestehend aus Kollagen Typ I, II, III, V, VI, IX, X, XI, XII, XIV, XVI, Chondroitinsulfat, Aggrecan, Keratansulfat, Hyaluronsäure, Proteoglycan 4, Cartilage oligomeric matrix protein (COMP), Fibromodulin, Procollagen II, Decorin, Anchorin, Hyaluronat, Biglycan, Thrombospondin, Fibronectin, Chondrocalcin, Alginat, Cellulose und Chitosan, Polymilchsäure (D und / oder L), Polyglycolsäure, deren CoPolymerisate, Polycaprolacton, Polyanhydride, Polyacetale und Polyketale, Polyethylenglycol, Poly(meth-)acrylate, Poly(glycidol), aromatische Polyester, PET, Polyoxacycline, Polyurethane, Polyvinyle, Polyvinylalkohole, Knorpelfragmente, Kollagenfibrillen und Mischungen davon. Die Zusammensetzung der Substanzen kann entsprechend der biochemischen Zusammensetzung der Gewebe, welche der Werkstoff nachbilden soll, beispielsweise unterschiedliche Knorpelgewebe oder Knochen, ausgewählt werden. Für die Herstellung von Modellmatrices ist das Polymer bevorzugt Alginat.

In einer bevorzugten Ausführungsform enthält die Substanz 0,5 bis 60 Gew.-% Polymer. Dadurch, dass die Bildung des Werkstoffs durch Erstarrung und Verfestigung erfolgt, können unterschiedliche Konzentrationen von Polymeren verwendet werden. Die Polymerkonzentrationen können dem natürlichen Gewebe, welches der Werkstoff nachbildet, entsprechen. Für eine hohe Festigkeit des Werkstoffs sind höhere Konzentrationen an Polymeren, vor allem an Strukturproteinen wie Kollagenen, bevorzugt. In einer ferner bevorzugten Ausführungsform enthält die Substanz daher 0,8 bis 10 Gew.-% Polymer, bevorzugt ca. 3 Gew.-% Polymer. In einer bevorzugten Ausführungsform enthält die erste und/oder eine weitere Substanz mindestens einen Stoff ausgewählt aus der Gruppe bestehend aus Keramiken, Salzen, Metalloxiden, Halbmetalloxiden, Nichtmetalloxide, Katalysatoren, Proteinen, Wachstumsfaktoren, medizinischen Wirkstoffen, Lipiden, Tensiden, Puffersubstanzen, und Mischungen davon. Entsprechend den Funktionen und physikalischen Eigenschaften, welche den Schichten des fertigen Werkstoffs zukommen, können eine oder mehrere Substanzen weitere Stoffe enthalten. In medizinische Werkstoffe können bevorzugt Stoffe mit medizinischer und/oder biologischer Wirkung integriert werden. Diese umfassen beispielsweise Antibiotika, Antiphlogistika, Antimykotika, β-Lactame wie Penicilline, Cephalosporine, Monobactame und Carbapeneme, Glykopeptide wie Vancomycine und Teicoplanine, Polyketide wie Tetracycline und Makrolide, Polypeptide wie Polymyxine, Bacitracin und Tyrothricin, Chinolone, Sulfonamide, Aminoglycoside, Streptomycine, Amphenicole, Aureomycine, nichtsteroidale Antiphlogistika, Glukokortikoide und Polyen-Antimykotika wie Amphotericin B. Nach Implantation oder Platzierung des Werkstoffs im Körper wirken diese Stoffe lokal, womit eine systemische Behandlung, wie beispielsweise durch Enzymhemmer oder Immunrepressiva, verhindert oder reduziert werden kann. Wird der Wirkstoff zu Implantationszwecken, beispielsweise als Trägermatrix bei Knorpeldefekten, verwendet, werden bevorzugt Wachstumsfaktoren, wie TGF, BMP, GDF, FGF, IGF, Annexin, MMP, PDGF, EGF, GMCSF, VEGF, HGF, Interleukine, NGF und CSF und/oder Stoffe, welche die Zellmigration fördern, in den Werkstoff integriert. Dadurch wird das Einwandern von Gewebezellen in den Werkstoff sowie die Bildung von extrazellulärer Matrix gefördert. Dies kann zur Integration des Werkstoffs ins Gewebe und auch zu einer vollständigen Wiederherstellung des Knorpels führen.

In einer bevorzugten Ausführungsform weist der sublimierbare Stoff einen Schmelzpunkt von ≤ 450°C, bevorzugt ≤ 90°C, ferner bevorzugt von -200°C bis +30°C, ferner bevorzugt von -100°C bis +20°C auf. Vorzugsweise ist der sublimierbare Stoff bei Raumtemperatur flüssig, was eine einfache Verarbeitung und Zubereitung der Substanzen erlaubt.

In einer bevorzugten Ausführungsform ist der sublimierbare Stoff ausgewählt aus der Gruppe bestehend aus wässrigen Lösungsmitteln, polaren Lösungsmitteln, unpolaren Lösungsmitteln, organischen Säuren, organischen Basen, mineralischen Säuren und mineralischen Basen. Der sublimierbare Stoff kann den bereits gelösten Bestandteilen der Substanz zugegeben werden oder, falls die übrigen Bestandteile der Substanz als Feststoff vorliegen, in dem sublimierbaren Stoff gelöst werden. In diesem Fall ist der sublimierbare Stoff bevorzugt ein Lösungsmittel, in welchem die übrigen Bestandteile der Substanz, beispielsweise Polymere gelöst werden. Insbesondere Strukturproteine sind nur schwer löslich, so dass sie bevorzugt in einer schwachen Säure, beispielsweise in 0,25 bis 5 M Essigsäure gelöst werden, welche auch als sublimierbarer Stoff dient. Der sublimierbare Stoff beeinflusst auch die Struktur der Poren im fertigen Werkstoff, weil diese durch die Kristalle des sublimierbaren Stoffs gebildet werden. Da sich die Kristallstrukturen verschiedener sublimierbarer Stoffe unterscheiden, kann durch die Wahl des sublimierbaren Stoffs die Form der Poren, insbesondere deren Größe und Verzweigung beeinflusst werden. In einer bevorzugten Ausführungsform ist der sublimierbare Stoff Wasser. Wasser ist als sublimierbarer Stoff besonders geeignet, da es ubiquitär verfügbar ist und bei vergleichsweise hohen Temperaturen bereits kristallisiert. Es ist daher nur eine geringe Kühlung der temperierbaren Körper notwendig, um die Substanzen, welche Wasser als sublimierbaren Stoff enthalten, zu erstarren. Dadurch wird das gesamte Verfahren energetisch vorteilhaft und kostengünstig.

In einer ferner bevorzugten Ausführungsform ist das polare Lösungsmittel ausgewählt aus der Gruppe bestehend aus Ethanol, Isopropanol, Aceton, Ether, Dimethylsulfoxid, Dimethylformamid, Tetrahydrofuran, N-Methyl-2-pyrrolidon, Chloroform, 1,4-Dioxan, Acrylnitril, und Acetonitril.

In einer ferner bevorzugten Ausführungsform ist das unpolare Lösungsmittel ausgewählt aus der Gruppe bestehend aus Benzol, Toluol, Methylenchlorid, Hexan, Heptan und Xylol.

In einer ferner bevorzugten Ausführungsform ist die organische Säure ausgewählt aus der Gruppe bestehend aus Carbonsäuren, Alkylcarbonsäuren, Essigsäure, Benzoesäure und Alkylsulfonsäuren.

In einer ferner bevorzugten Ausführungsform ist die mineralische Säure ausgewählt aus der Gruppe bestehend aus Natronlauge, Kaliumhydroxid, Kalkwasser, Phosphorsäure und Salzsäure.

In einer bevorzugten Ausführungsform weist die erste Substanz und mindestens eine weitere Substanz, bevorzugt jede weitere Substanz, den gleichen sublimierbaren Stoff auf. Beinhalten zwei Substanzen benachbarter Schichten den gleichen sublimierbaren Stoff, verbinden sich die Kristalle an der Oberfläche der ersten, bereits erstarrten Schicht, mit den noch flüssigen Molekülen des sublimierbaren Stoffs in der zweiten Substanz, wenn diese auf die bereits erstarrte Schicht aufgetragen wird. Dies fördert das Einwachsen der Kristalle, welche über die erste bereits starre Schicht hinausragen, in die nächste Schicht. Außerdem gleichen sich die Kristallstrukturen in den Schichten, wenn der gleiche sublimierbare Stoff verwendet wird, wodurch nach der Sublimation alle Schichten gleichförmige Poren aufweisen.

In einer bevorzugten Ausführungsform weist mindestens einer der temperierbaren oder isolierenden Körper oder das Behältnis eine Mikrostrukturierung auf. Der Begriff "Mikrostrukturierung" bezeichnet eine Strukturierung an der Oberfläche eines temperierbaren oder isolierenden Körpers oder eines Behältnisses in Form von Erhebungen und/oder Vertiefungen im Abstand weniger Mikrometer. Diese Strukturierung initiiert die Bildung von Kristallisationskeimen, wodurch gesteuert werden kann, an welchen Stellen die Kristallisation bzw. Erstarrung zuerst einsetzt. Von den Kristallisationskeimen aus breitet sich die Erstarrung weiter aus, wodurch die räumliche Orientierung der Kristalle und damit der späteren Poren gesteuert werden kann. Durch Mikrostrukturierungen in Form von konzentrischen Kreisen, Bögen, Wellen, oder Linien kann eine entsprechende Anordnung der Poren innerhalb des Werkstoffs erreicht werden. Die Mikrostrukturierung kann aber auch dazu dienen, die Oberfläche des Werkstoffs zu gestalten, beispielsweise um die Adhäsion von Zellen an den Werkstoff zu fördern. Dient die Mikrostrukturierung der Steuerung der Kristallisation, wird sie bevorzugt auf dem temperierbaren Körper, oder auf das Behältnis in welches die Substanzen eingebracht werden, aufgebracht, der/das am kältesten Punkt des Temperaturgradienten angeordnet ist.

In einer bevorzugten Ausführungsform erfolgt das Sublimieren bei einem Druck von ≤ 6 mbar und einer Temperatur von ≤ 0°C, bevorzugt bei einem Druck von 10 µbar bis 1 mbar und einer Temperatur von -80°C bis -20°C , ferner bevorzugt bei einem Druck von 50 µbar bis 90 µbar und einer Temperatur von -60°C bis -30°C . Durch das Sublimieren wird der in den erstarrten Schichten auskristallisierte sublimierbare Stoff von seiner Festphase in die Gasphase überführt. Das entstehende Gas wird abgesaugt, so dass anstelle der Kristalle des sublimierbaren Stoffs Hohlkörper, in Form von Poren, übrig bleiben. Der Sublimationsdruck und die Sublimationstemperatur richten sich nach dem verwendeten sublimierbaren Stoff und können aus Temperatur-Phasen-Diagrammen entnommen werden. Die Sublimation von Wasser erfolgt bei unter 6 mbar und unter 0°C. Diese Sublimationsparameter sind auch für wässrige Lösungen von Säuren oder Basen, beispielsweise für 0,25 bis 5 M Essigsäure geeignet.

In einer bevorzugten Ausführungsform umfasst das Verfahren ferner den Schritt Anordnen einer Schicht funktionalisierter Polymerfasern auf der Trägermatrix als äußerste Schicht. Die Schicht funktionalisierter Polymerfasern kann als reibungsmindernde Oberfläche, oder abschließende Membran fungieren. Die Funktion als reibungsmindernde Oberfläche kann durch die Verwendung von Lubricinen verstärkt werden. Durch eine Faserausrichtung parallel zur Werkstoffoberfläche können dort auftretende Scherkräfte besser von dem Werkstoff verarbeitet werden. Sie kann durch funktionalisierte Polymerfasern (z. B. Kollagen Typ I, II, III, V, VI, IX, X, XI, XII, XIV, XVI, oder lineare, verzweigte und sternförmige Polymere auf Basis von Polyethylenglykol) erzeugt werden. Die Schicht funktionalisierter Polymerfasern kann mittels elektrostatischem Verspinnen, vor oder nach dessen Verfestigung, auf den Werkstoff aufgebracht werden (Grafahrend et al., 2010).

In einem weiteren Aspekt betrifft die Offenbarung einen mehrschichtigen Werkstoff, der durch das erfindungsgemäße Verfahren herstellbar ist. Der durch das Verfahren hergestellte Werkstoff umfasst mehrere Schichten, die sich in ihrer Funktionalität, ihrer Zusammensetzung und/oder ihren physikalischen Eigenschaften unterscheiden. Die verschiedenen Schichten sind zu einem Monolithen, das heißt zu einem Stück bzw. einstückig, vereinigt, so dass eine monolithische Struktur mit anisotropen Poren entsteht. Auf diese Weise vereint der Werkstoff mehrere unterschiedliche Bereiche und kann so komplexe Anforderungen, wie sie vor allem im medizinischen Bereich auftreten, erfüllen. Beispielsweise kann mit dem Werkstoff der schichtweise Aufbau einer natürlichen Knochen-Knorpelstruktur nachgebildet werden (Figur 2). Darüber hinaus zeichnet sich der Werkstoff durch eine alle Schichten durchdringende anisotrope Porenstruktur aus, die ihm spezifische physikalische und biologische Eigenschaften verleiht. Durch die anisotrope Porenstruktur wird die Stabilität des Werkstoffs verbessert, und indem die Poren durch alle Schichten kontinuierlich hindurchragen, die Grenzen der Schichten also überschreiten, wird der Zusammenhalt der einzelnen Schichten gefördert und eine Delamination verhindert. Die Poren ermöglichen zudem das Eindringen von Stoffen bis in das Innere des Werkstoffs. Auch ist durch die Poren eine Besiedelung des Werkstoffs mit Zellen möglich, da die durchgehenden Poren ein Einwandern der Zellen bis in das Innere des Werkstoffs ermöglichen. Dies ist vor allem für die Verwendung des Werkstoffs als Knochen/Knorpelersatz bei Gelenkdefekten von Bedeutung, da die Besiedelung des Werkstoffs nach Implantation mit Chondrozyten zum Wiederaufbau des Knorpels und zur vollständigen Integration des Implantats beiträgt. Darüber hinaus ist der Werkstoff auch besonders zur Kultivierung von Zellen geeignet, denn er stellt eine dreidimensionale Struktur bereit, die im Gegensatz zu herkömmlichen zweidimensionalen Kultivierungsgefäßen die natürliche Umgebung der Zellen imitiert. Durch die anisotrope Porenstruktur kann ebenfalls ein adäquater Austausch von Nähr- und Schadstoffen erfolgen.

In einer bevorzugten Ausführungsform weisen die Poren eine kolumnare, lamellare und/oder dendritische Struktur auf. Die Struktur der Poren wird durch die Art des sublimierbaren Stoffs, der sonstigen Bestandteile der Substanzen, sowie durch die Temperaturen der temperierbaren Körper bzw. den Temperaturgradienten bestimmt. Unter einer lamellaren Morphologie ist hierbei eine in Form von Lamellen angeordnete, erstarrte Phase der sublimierbaren Zusätze zu verstehen. Eine kolumnare Morphologie entspricht einer stab- oder prismaförmigen Erscheinungsform der erstarrten Phase der sublimierbaren Zusätze. Ragen aus Seitenflächen einer kolumnaren Morphologie Verästelungen oder Verzweigungen heraus, so handelt es sich um eine dendritisch erstarrte Phase der sublimierbaren Zusätze. Im Gegensatz zu kolumnaren, lamellaren und dentritischen Kristallen weisen equiaxiale Kristalle eine kugelförmige dendritische Struktur auf. Kolumnare Porenstrukturen erhöhen die Stabilität des Werkstoffs, so dass dieser widerstandsfähiger gegen Verformungen ist. Lamellare Poren erleichtern hingegen die Besiedelung des Werkstoffs mit Zellen.

Die mindestens zwei Schichten weisen eine unterschiedliche Zusammensetzung auf. Werkstoffe, die Schichten oder Bereiche mit unterschiedlichen Zusammensetzungen aufweisen, können mehrfache Funktionalitäten erfüllen. Dies ist insbesondere für medizinische Werkstoffe von Bedeutung, die in natürliches Gewebe integriert werden. Die meisten Gewebe im menschlichen oder tierischen Körper vereinen unterschiedliche Eigenschaften oder führen unterschiedliche Funktionen aus, indem sie verschiedene Bereiche unterschiedlicher zellulären oder extrazellulären Zusammensetzung besitzen. Dies gilt beispielsweise für die extrazelluläre Matrix, die einerseits die Organe stützt und zusammenhält, aber auch Zelladhäsion steuert und Wasser speichert. Indem ein medizinischer Werkstoff mehrere Schichten unterschiedlicher chemischer Zusammensetzungen vereint, kann er die Mehrfachfunktionalität natürlicher Gewebe, beispielsweise der natürlichen extrazellulären Matrix, nachbilden.

In einer bevorzugten Ausführungsform weisen die mindestens zwei Schichten unabhängig voneinander mindestens ein Polymer auf. Polymere sind bevorzugte Bestandteile der Schichten, da sie durch intermolekulare Verbindungen stabile Strukturen und Netzwerke ausbilden. Vorzugsweise ist das Polymer ein natives Polymer.

In einer bevorzugten Ausführungsform weisen die Poren einen Durchmesser von 20 µm bis 380 µm, bevorzugt von 50 µm bis 120 µm, ferner bevorzugt von ca. 80 µm auf. Um eine effiziente Besiedelung des Werkstoffs durch Zellen zu gewährleisten sollten die Poren einen Durchmesser von mindestens 20 µm aufweisen. Insbesondere für Werkstoffe, die als (Knochen-) Knorpelersatz verwendet werden, sind Poren mit einem größeren Durchmesser, beispielsweise von 60 µm bis 100 µm vorteilhaft, weil sie nicht nur die Migration der Zellen ins Innere des Werkstoffs ermöglichen, sondern auch Raum für die Bildung extrazellulärer Matrix im Inneren des Werkstoffs lassen. Interessanterweise ist auch eine effiziente Besiedelung von Werkstoffen mit relativ schmalen Porenkammern, beispielsweise mit Durchmessern von 20 µm bis 50 µm möglich, wenn die Zusammensetzung der Werkstoffe der natürlichen extrazellulären Matrix besonders ähnlich ist.

In einem weiteren Aspekt betrifft die Offenbarung einen medizinischen Werkstoff mit mindestens zwei verschiedenen Schichten, in dem sich Poren anisotrop durch mindestens zwei Schichten des Werkstoffs erstrecken. Der medizinische Werkstoff kann durch das erfindungsgemäße Verfahren hergestellt werden und in den oben für einen mehrschichtigen Werkstoff beschriebenen bevorzugten Ausführungsformen vorliegen.

In einem weiteren Aspekt betrifft die Offenbarung die Verwendung eines mehrschichtigen Werkstoffs als medizinischen Werkstoff, in dem sich Poren anisotrop durch mindestens zwei Schichten des Werkstoffs erstre-cken. Der Werkstoff kann durch das erfindungsgemäße Verfahren hergestellt werden und in den oben für einen mehrschichtigen Werkstoff beschriebenen bevorzugten Ausführungsformen vorliegen.

In einem weiteren Aspekt betrifft die Offenbarung die Verwendung eines mehrschichtigen Werkstoffs als medizinische Trägermatrix, in dem sich Poren anisotrop durch mindestens zwei Schichten des Werkstoffs erstrecken. Durch die anisotrope Porenstruktur, welche sich über die Grenzen der Schichten hinaus durch den gesamten Werkstoff erstreckt, ist dieser als medizinische Trägermatrix besonders geeignet, weil eine ganzheitliche Besiedelung des Werkstoffs durch Zellen möglich ist.

In einem weiteren Aspekt betrifft die Offenbarung die Verwendung eines mehrschichtigen Werkstoffs als (osteo)chondrale Trägermatrix, in dem sich Poren anisotrop durch mindestens zwei Schichten des Werkstoffs erstrecken. Native Knorpelstrukturen zeichnen sich durch ihren mehrschichtigen Aufbau aus. Indem der Werkstoff mehrere unterschiedliche Schichten vereint, die zudem von anisotropen Poren durchzogen sind, entspricht er nicht nur im Aufbau der natürlichen (osteo)chrondralen Matrix, sondern kann auch hinsichtlich der Zusammensetzungen der Schichten dieser nachgebildet werden.

In einem weiteren Aspekt betrifft die Offenbarug die Verwendung eines mehrschichtigen Werkstoffs als Meniskus-Trägermatrix, dadurch gekennzeichnet, dass sich Poren anisotrop durch mindestens zwei Schichten des Werkstoffs erstrecken. Durch das erfindungsgemäße Verfahren kann der mehrschichtige poröse Werkstoff in Form eines Meniskus hergestellt werden. Die Struktur eines natürlichen Meniskus zeichnet sich durch die unterschiedliche Zusammensetzung des äußeren und des inneren Bereichs des Meniskus und durch die parallel zum peripheren Rand verlaufende Anordnung der Kollagenfibrillen aus. Diese Struktur wird durch die unterschiedlichen Schichten und die Orientierung der Poren in der Trägermatrix nachgebildet. So entstehen durch das Überwachsen zentraler Kristalle durch benachbarte Kristalle während des Erstarrens bogenförmige, oder schräge Strukturen. Eine solche Struktur kann beispielsweise erhalten werden, indem Substanzen verwendet werden, die eine relative hohe Viskosität haben und vortemperiert sind. Die Temperierung der Substanzen sollte nahe dem Temperaturniveau des wärmeren temperierbaren Körpers liegen, beispielsweise 0,5°C bis 5°C über dem Temperaturniveau des wärmeren temperierbaren Körpers. Auf diese Weise werden nach Sublimation bogenförmige, oder schräge Poren in einem Winkel von bis zu 90° zu dem Temperaturgradienten erhalten.

Ein weiterer Aspekt der Offenbarung betrifft die Verwendung eines mehrschichtigen Werkstoffs als Bandscheiben-Trägermatrix, in dem sich Poren anisotrop durch mindestens zwei Schichten des Werkstoffs erstrecken. Durch das erfindungsgemäße Verfahren können sowohl Werkstoffe mit übereinander angeordneten Schichten, als auch solche mit nebeneinander oder konzentrisch angeordneten Schichten, hergestellt werden. Letztere sind vor allem für eine Bandscheiben-Trägermatrix geeignet. Angelehnt an die natürliche Bandscheibe, besitzt eine solche Trägermatrix eine innere Schicht, die dem Nucleus pulposus entspricht, sowie darum konzentrisch angeordnet Schichten, die dem inneren und dem äußeren Anulus fibrosus entsprechen. Durch die Verwendung von Substanzen mit unterschiedlichen Zusammensetzungen können die jeweiligen Schichten den physikalischen Eigenschaften der unterschiedlichen Bereiche einer Bandscheibe angepasst werden. Um die Porosität der natürlichen Bandscheibe nachzubilden, besitzt die innere Schicht isotrope Poren, wohingegen die Schichten des inneren und äußeren Anulus fibrosus anisotrope Poren aufweisen. Dies fördert die Stabilität der Matrix und ihre Integration in natürliches Gewebe.

In einem weiteren Aspekt betrifft die Offenbarung ein Verfahren zur Herstellung einer mehrschichtigen (osteo)chondralen Trägermatrix, umfassend die Schritte Bereitstellen eines Temperaturgradienten durch zwei einander gegenüber liegend angeordnete temperierbare Körper; Anordnen und Erstarren einer ersten Substanz, die mindestens ein Polymer, mindestens ein Glycosaminoglycan und mindestens einen sublimierbaren Stoff enthält, in dem Temperaturgradienten, um eine mittlere Chondralzone zu bilden; Anordnen und Erstarren einer zweiten Substanz, die mindestens ein Polymer, mindestens ein Glycosaminoglycan und mindestens einen sublimierbaren Stoff enthält, in dem Temperaturgradienten, um eine untere Chondralzone zu bilden, die unmittelbar an die mittlere Chondralzone anschließt; optional Anordnen und Erstarren einer dritten Substanz, die mindestens ein Polymer, mindestens ein Erdalkaliphosphat und mindestens einen sublimierbaren Stoff enthält, in dem Temperaturgradienten, um eine Subchondralzone zu bilden, die unmittelbar an die untere Chondralzone anschließt; Sublimieren des Stoffs, und Verfestigen der Schichten.

Durch das Verfahren können sowohl mehrschichtige chrondrale Trägermatrices (ohne Subchondralzone) als auch osteochondrale Trägermatrices (mit Subchondralzone) hergestellt werden. Die Trägermatrices sind insbesondere zur Behandlung von Knorpel bzw. Knochen-Knorpeldefekten geeignet, da die chemische Zusammensetzung der einzelnen Schichten den natürlichen Schichten des Knorpels, das heißt der oberen Chondralzone, der unteren Chondralzone und der Subchondralzone (Figur 2) entsprechen. Entsprechend den Bestandteilen der natürlichen Knorpelmatrix beinhalten die Substanzen zur Bildung der oberen und unteren Chondralzone mindestens ein Polymer und mindestens ein Glycosaminoglycan und die Substanz zur Bildung der Subchondralzone mindestens ein Polymer und mindestens ein Erdalkaliphosphat. Das Gerüst der extrazellulären Matrix des nativen Gelenkknorpels besteht im Wesentlichen aus einer Calciumphosphat-haltigen Subchondralzone (Übergangszone zum Knochen), aus der sich Kollagenfasern erheben (Figur 2). Diese sind in tiefer gelegenen Knorpelarealen (d) normal zur Knochenoberfläche orientiert, in mittleren Arealen (m) wandelt sich dies zu einem verschränkten Faserarrangement, welches wiederum in Nähe zur Oberfläche (s) parallel zu dieser verläuft. Zusammen mit der Kollagenfaserstruktur ändert sich auch das Erscheinungsbild der Knorpelzellen (Chondrozyten) und somit auch die, deren Zellverbände (Chondrone). Während die Chondrozyten in den unteren (d) und mittleren (m) Knorpelarealen rund geformt sind und kolumnare Chondrone ausbilden, so weisen sie in der oberen Knorpelzone (s) eine flache Gestalt auf, und sind in Form von horizontal verlaufenden Chondronen zusammengelagert.

Nach der Sublimation wird die monolithische Matrix verfestigt, beispielsweise indem sie mittels aktivierten Carbondiimiden, Isocyanaten, komplexbildenden Ionen, nicht-enzymatischer Glykation oder Glutaraldehyd nasschemisch vernetzt wird. Bevorzugt erfolgt die Vernetzung mittels N-Hydroxysuccinimid und 1-Ethyl-3-(3-dimethylaminopropyl)carbodiimid bei Pufferung durch 2-Morpholinoethansulfonsäure. Bei dieser zellverträglichen "zero-length"-Vernetzung entstehen kovalente Bindungen zwischen den Kollagenen, ohne dass weitere Stoffe eingebaut werden. Ferner bevorzugt wird die Trägermatrix vor der nasschemischen Vernetzung dehydrothermal vorvernetzt, beispielsweise bei Drücken zwischen 1*10⁻⁶ mbar und 100 mbar und Temperaturen zwischen 50 °C und 200° C.

In einer bevorzugten Ausführungsform umfasst das Verfahren ferner den Schritt Anordnen einer Schicht funktionalisierte Polymerfasern auf der Trägermatrix. Die Schicht funktionalisierte Polymerfasern bildet eine reibungsmindernde Oberfläche, die der Gelenkinnenseite des nativen Knorpels entspricht. Sie kann durch funktionalisierte Polymerfasern (z. B. Kollagen Typ I, II, III, V, VI, IX, X, XI, XII, XIV, XVI, oder lineare, verzweigte und sternförmige Polymere auf Basis von Polyethylenglykol) erzeugt werden, welche eine abschließende Gleitschicht (CS) ausbilden. Die Schicht funktionalisierter Polymerfasern kann mittles elektrostatischem Verspinnen, vor oder nach dessen Verfestigung, auf den Werkstoff aufgebracht werden.

Auf Grund der gerichteten Erstarrung ist die mehrschichtige chondrale Trägermatrix von anisotropen Poren durchzogen. Dadurch wird eine effiziente Zellmigration ins Innere der Matrix gewährleistet, welche zur vollständigen Integration der Trägermatrix in den defekten Knorpel und deren Funktionalität entscheidend beiträgt. Eine weitere Verbesserung der Zellbesiedelung kann durch eine Matrixkompression erhalten werden. Hierunter ist eine, durch äußeren mechanischen Druck hervorgerufene, Verformung der Trägerstruktur zu verstehen, welche aufgrund der auftretenden Kapillarkräfte einen Sogeffekt auf die Zellen ausübt und diese in das Innere der Matrix hineinzieht.

In einer bevorzugten Ausführungsform ist das mindestens eine Polymer, das in der ersten, zweiten und/oder dritten Substanz enthalten ist, unabhängig voneinander ein Kollagen, ausgewählt aus der Gruppe bestehend aus Kollagen Typ I, II, III, V, VI, IX, X, XI, XII, XIV, XVI, bevorzugt Kollagen Typ I oder Typ II. Kollagene sind die natürlichen Strukturproteine des Knorpels und bilden den überwiegenden Feststoffanteil aller Zonen des natürlichen Knorpels. Für die Herstellung der Substanzen der Vorstufen der einzelnen Schichten sind Kollagene, die aus Säugetieren gewonnen und aufgereinigt werden geeignet. Speziell geeignet sind Kollagene, die durch enzymatische Spaltung aufgeschlossen wurden, sowie zerkleinerte gereinigte Knorpelfragmente und Kollagenfibrillen. Bevorzugt enthalten die Substanzen 0,5 bis 60 Gew.-%, ferner bevorzugt 0,8 bis 10 Gew.-% Kollagene.

In einer bevorzugten Ausführungsform ist das mindestens eine Glycosaminoglycan, das in der ersten und/oder zweiten Substanz enthalten ist, unabhängig voneinander ausgewählt aus der Gruppe bestehend aus Chondroitinsulfat, Aggrecan, Keratansulfat, Hyaluronsäure, Proteoglycan 4, Cartilage oligomeric matrix protein (COMP), Fibromodulin, Procollagen II, Decorin, Anchorin, Hyaluronat, Biglycan, Thrombospondin, Fibronectin, und Chondrocalcin. Bevorzugt weist der Trockenmasseanteil der Substanzen einen Anteil an Glycosaminoglycanen von 1 bis 35 % auf, wobei ein höherer Anteil an Glycosaminoglycanen zu einer erhöhten Widerstandsfähigkeit gegen Kompressionen der Trägermatrix führt.

In einer bevorzugten Ausführungsform ist das mindestens eine Erdalkaliphosphat, das in der dritten Substanz enthalten ist, ein Calciumphosphat oder ein Magnesiumphosphat, bevorzugt ausgewählt aus der Gruppe bestehend aus Bruschit, Monetit, Hydroxylapatit, α-Tricalciumphosphat, β-Tricalciumphosphat Whitlockit, Struvit, Newberit, und Farringtonit. Höhere Anteile an Erdalkaliphosphaten erhöhen die Widerstandsfähigkeit der Trägermatrix gegen Kompressionen. Die Erdalkaliphosphate können in Form von Erdalkaliphosphatkristalliten, Erdalkaliphosphat-Substraten oder in Form von Kompositwerkstoffen vorliegen. Erdalkaliphosphate können beispielsweise durch Zementreaktion, durch 3D Rapid Prototyping von Erdalkaliphosphatpulvern oder deren Ausgangsstoffen, oder durch gerichtete Erstarrung mit anschließender Sinterung von erdalkaliphosphathaltigen Substanzen gewonnen werden. Erdalkaliphosphatkompositwerkstoffe lassen sich unter anderem durch Bioplotting eines Gemisches aus Polymeren und Erdalkaliphosphaten, oder Stoffen, welche zu Erdalkaliphosphaten reagieren, erzeugen.

In einer bevorzugten Ausführungsform enthält die erste, zweite und/oder dritte Substanz, unabhängig voneinander, Antibiotika und/oder Wachstumsfaktoren, wie beispielsweise TGF, BMP, GDF, IGF, Annexin und MMP. Indem das gesamte Herstellungsverfahren ausschließlich bei niedrigen Temperaturen stattfindet, ist es besonders geeignet um medizinische Wirkstoffe, die regelmäßig temperaturempfindlich sind, in die einzelnen Schichten der Trägermatrix zu integrieren. Die Wirkstoffe können auch in eingekapselter Form innerhalb der Substanzen vorliegen.

In einer bevorzugten Ausführungsform ist der sublimierbare Stoff Essigsäure. Essigsäure ist als sublimierbarer Stoff besonders bevorzugt, da sie als schwache Säure Proteinstrukturen weniger stark beeinträchtigt als starke Säuren, sich deren Rückstände gut durch Sublimation entfernen lassen und eine vergleichsweise gute Zellverträglichkeit aufweisen.. Je höher die Konzentration am zu lösenden Kollagen, desto höhere Molaritäten von Essigsäure werden eingesetzt. In einer besonders bevorzugten Ausführungsform enthält der sublimierbare Stoff daher 0,25 - 4 M Essigsäure, bevorzugt 0,5 - 3 M Essigsäure, ferner bevorzugt 0,5 M Essigsäure.

In einer bevorzugten Ausführungsform enthält die erste, zweite und/oder dritte Substanz 0,5 - 60 Gew.-% Polymer, bevorzugt 0,8 - 10 Gew.-% Polymer, ferner bevorzugt 1 - 5 Gew.-% Polymer. Höhere Polymeranteile führen zu einer stabileren Struktur der Trägermatrix und sind daher insbesondere zur Verwendung in Gelenken, welche durch das Körpergewicht belastet werden, beispielsweise Kniegelenke, bevorzugt.

In einer bevorzugten Ausführungsform beträgt der Temperaturgradient zur Herstellung von (osteo)chondralen Trägermatrices zwischen 5 und 10 K/mm, bevorzugt 8 K/mm.

Die Trägermatrix ist insbesondere geeignet zur Behandlung chondraler und osteochondraler Defekte, sowohl in Form von matrixgekoppelter autologer Chondrozytentransplantation (MACT), als auch als zum Zeitpunkt der Implantation zellfreie Matrix. Außerdem kann die erfindungsgemäße Trägermatrix zur Zellkultivierung eingesetzt werden.

In einem weiteren Aspekt betrifft die Offenbarung ein Verfahren zur Herstellung einer mehrschichtigen Meniskus-Trägermatrix, umfassend die Schritte Bereitstellen eines Temperaturgradienten durch zwei einander gegenüber liegend angeordnete temperierbare Körper; Anordnen und Erstarren einer ersten Substanz, die Kollagen I, mindestens ein Glycosaminoglycan und mindestens einen sublimierbaren Stoff enthält, in dem Temperaturgradienten, um einen äußeren Meniskusbereich zu bilden; Anordnen und Erstarren einer zweiten Substanz, die Kollagen I, Kollagen II, mindestens ein Glycosaminoglycan und mindestens einen sublimierbaren Stoff enthält, in dem Temperaturgradienten, um einen inneren Meniskusbereich zu bilden, der unmittelbar an den äußeren Meniskusbereich anschließt; Sublimieren des Stoffs und Verfestigen der Trägermatrix.

Die durch das Verfahren erhaltene Trägermatrix entspricht sowohl in ihrer Zusammensetzung als auch in ihrer Mikrostruktur dem natürlichen Meniskus. Zum Erstarren der Substanzen werden temperierbare und isolierende Körper oder ein Behältnis verwendet, die/das der Negativform eines Meniskus entsprechen/entspricht (Figur 5 b). Dadurch wird die meniskusartige äußere Erscheinungsform der Trägermatrix erhalten (Figur 5 c). Durch die unterschiedlichen Schichten der Trägermatrix wird der äußere und innere Meniskusbereich nachgebildet (Figur 5 a). Die Orientierung der Poren in der Trägermatrix bildet die parallel zum peripheren Rand verlaufende Anordnung der Kollagenfibrillen des natürlichen Meniskus nach.

Nach der Sublimation wird die monolithische Matrix verfestigt, beispielsweise indem sie mittels aktivierten Carbondiimiden, Isocyanaten, komplexbildenden Ionen, nicht-enzymatischer Glykation oder Glutaraldehyd nasschemisch vernetzt wird. Bevorzugt erfolgt die Vernetzung mittels N-Hydroxysuccinimid und 1-Ethyl-3-(3-dimethylaminopropyl)carbodiimid bei Pufferung durch 2-Morpholinoethansulfonsäure. Bei dieser zellverträglichen "zero-length"-Vernetzung entstehen kovalente Bindungen zwischen den Kollagenen, ohne dass weitere Stoffe eingebaut werden. Ferner bevorzugt wird die Trägermatrix vor der nasschemischen Vernetzung dehydrothermal vorvernetzt, beispielsweise bei Drücken zwischen 1*10⁻⁶ mbar und 100 mbar und Temperaturen zwischen 50 °C und 200° C.

In einer bevorzugten Ausführungsform umfasst das Verfahren ferner den Schritt Anordnen einer Schicht funktionalisierte Polymerfasern auf der Trägermatrix. Diese Polymerfasern entsprechen einer reibungsmindernden äußeren Schicht des natürlichen Meniskus und bildet eine abschließende Gleitschicht auf der Oberfläche. Die Schicht funktionalisierter Polymerfasern kann mittles elektrostatischem Verspinnen, vor oder nach dessen Verfestigung, auf den Werkstoff aufgebracht werden.

In einer bevorzugten Ausführungsform beträgt der Temperaturgradient zur Herstellung von Meniskus-Trägermatrices zwischen 3 und 8 K/mm, bevorzugt 5 K/mm.

Die Trägermatrix ist geeignet zur Behandlung von Meniskusdefekten. Das erfindungsgemäße Verfahren ermöglicht es, einen Meniskusersatz entsprechend den individuellen Gegebenheiten der Gelenke des Patienten herzustellen. Dazu werden die temperierbaren und isolierenden Körper oder ein Behältnis, das die Form der Trägermatrix bestimmt, nach dreidimensionalen Rekonstruktionen des Meniskusdefekts des Patienten angefertigt. Diese Form wird verwendet um eine passgenaue Trägermatrix herzustellen.

In einem weiteren Aspekt betrifft die Offenbarung ein Verfahren zur Herstellung einer mehrschichtigen Bandscheiben-Trägermatrix, umfassend die Schritte Bereitstellen eines Temperaturgradienten durch zwei einander gegenüber liegend angeordnete temperierbare Körper; Anordnen einer ersten Schicht, die einen Kern bildet und aus einer ersten Substanz, die mindestens ein Polymer, mindestens ein Glycosaminoglycan und mindestens einen sublimierbaren Stoff enthält; gebildet ist; Anordnen und Erstarren einer zweiten Substanz, die mindestens ein Polymer, mindestens ein Glycosaminoglycan und mindestens einen sublimierbaren Stoff enthält, in dem Temperaturgradienten, um eine innere Schicht zu bilden, die unmittelbar an den Kern anschließt; Anordnen und Erstarren einer dritten Substanz, die mindestens ein Polymer, mindestens ein Glycosaminoglycan und mindestens einen sublimierbaren Stoff enthält, in dem Temperaturgradienten, um eine äußere Schicht zu bilden, die unmittelbar an die innere Schicht anschließt; Sublimieren des Stoffs und Verfestigen der Schichten.

Die natürliche Bandscheibenstruktur besteht im Wesentlichen aus einem inneren Kern, dem Nucleus pulposus, welcher von faserigen Lamellen umschlossen wird, die den Anulus fibrosus bilden (Figur 7 a). Der Nucleus pulposus (NP) ist strukturell und mechanisch isotrop und enthält ein Proteoglykan-haltiges Netzwerk von Kollagen Typ II. Der Anulus fibrosus besteht aus einer Vielzahl von Lamellen, welche im äußeren Teil (äußerer Anulus fibrosus, oAF) aus Kollagen Typ I und im inneren Teil (innerer Anulus fibrosus, iAF) aus Kollagen Typ II bestehen. Zusammen mit der inneren Bandscheibenstruktur ändert sich auch das Erscheinungsbild der Zellen, die in der Bandscheibe vorkommen. Während die Notochordal-Zellen im Nucleus pulposus rund geformt sind, so sind die Zellen im inneren Anulus fibrosus chondrozytär. Die Zellen im äußeren Anulus fibrosus werden als Fibrochondrozyten beschrieben. Entsprechend dem nativen Gewebe weist die Trägermatrix Bestandteile der extrazellulären Knorpelmatrix auf, welche zu einer biomimetischen, monolithischen Trägermatrix vereinigt sind. Ein ungerichtetes Netzwerk befindet sich im Kern der Matrix und entspricht dem Nucleus pulposus. Dieses ist von zwei Schichten unterschiedlicher Zusammensetzung umgeben, die einen lamellaren Aufbau besitzen und dem inneren und äußeren Anulus fibrosus entsprechen.

Nach der Sublimation wird die monolithische Matrix verfestigt, beispielsweise indem sie mittels aktivierten Carbondiimiden, Isocyanaten, komplexbildenden Ionen, nicht-enzymatischer Glykation oder Glutaraldehyd nasschemisch vernetzt wird. Bevorzugt erfolgt die Vernetzung mittels N-Hydroxysuccinimid und 1-Ethyl-3-(3-dimethylaminopropyl)carbodiimid bei Pufferung durch 2-Morpholinoethansulfonsäure. Bei dieser zellverträglichen "zero-length"-Vernetzung entstehen kovalente Bindungen zwischen den Kollagenen, ohne dass weitere Stoffe eingebaut werden. Ferner bevorzugt wird die Trägermatrix vor der nasschemischen Vernetzung dehydrothermal vorvernetzt, beispielsweise bei Drücken zwischen 1*10⁻⁶ mbar und 100 mbar und Temperaturen zwischen 50 °C und 200° C.

In einer bevorzugten Ausführungsform umfasst das Verfahren ferner den Schritt Anordnen einer Schicht funktionalisierter Polymerfasern auf der Trägermatrix. Diese Polymerfasern entsprechen der äußeren Membran, welche die natürliche Bandscheibe umgibt. Die Schicht funktionalisierter Polymerfasern kann mittles elektrostatischem Verspinnen, vor oder nach dessen Verfestigung, auf den Werkstoff aufgebracht werden.

In einer bevorzugten Ausführungsform ist das mindestens eine Polymer, das in der ersten, zweiten und/oder dritten Substanz enthalten ist, unabhängig voneinander ein Kollagen, ausgewählt aus der Gruppe bestehend aus Kollagen Kollagen Typ I, II, III, V, VI, IX, X, XI, XII, XIV, XVI, Cellulose, Chitosan, Polymilchsäure (D und / oder L) bzw. Polyglycolsäure, Polycaprolacton, und Polyethylenglycol, bevorzugt Kollagen Typ I oder Typ II. Kollagene sind die natürlichen Strukturproteine des Knorpels und bilden den überwiegenden Feststoffanteil aller Zonen des natürlichen Knorpels. Für die Herstellung der Substanzen der Vorstufen der einzelnen Schichten sind Kollagene, die aus Säugetieren gewonnen und aufgereinigt werden geeignet. Speziell geeignet sind Kollagene, die durch enzymatische Spaltung aufgeschlossen wurden, sowie zerkleinerte gereinigte Knorpelfragmente und Kollagenfibrillen. In einer ferner bevorzugten Ausführungsform enthält die erste Substanz Kollagen Typ II, die zweite Substanz Kollagen Typ II und die dritte Substanz Kollagen Typ I.

In einer bevorzugten Ausführungsform ist das mindestens eine Glycosaminoglycan, das in der ersten, zweiten und/oder dritten Substanz enthalten ist, unabhängig voneinander ausgewählt aus der Gruppe bestehend aus Chondroitinsulfat, Aggrecan, Keratansulfat, Hyaluronsäure, Proteoglycan 4, Cartilage oligomeric matrix protein (COMP), Fibromodulin, Procollagen II, Decorin, Anchorin, Hyaluronat, Biglycan, Thrombospondin, Fibronectin und Chondrocalcin. Bevorzugt weist der Trockenmasseanteil der Substanzen zu Herstellung von Bandscheiben-Trägermatrices einen Anteil an Glycosaminoglycanen von 10 bis 55 % auf, wobei ein höherer Anteil an Glycosaminoglycanen zu einer erhöhten Widerstandsfähigkeit der Trägermatrix gegen Kompressionen führt.

In einer bevorzugten Ausführungsform enthält die erste, zweite und/oder dritte Substanz, unabhängig voneinander, Antibiotika und/oder Wachstumsfaktoren, beispielsweise TGF, BMP, GDF, IGF, Annexin und MMP. Indem das gesamte Herstellungsverfahren ausschließlich bei niedrigen Temperaturen stattfindet, ist es besonders geeignet um medizinische Wirkstoffe, die regelmäßig temperaturempfindlich sind, in den einzelnen Schichten der Trägermatrix zu integrieren. Die Wirkstoffe können auch in eingekapselter Form innerhalb der Substanzen vorliegen.

In einer bevorzugten Ausführungsform ist der sublimierbare Stoff Essigsäure. Essigsäure ist als sublimierbarer Stoff besonders bevorzugt, da sie als schwache Säure Proteinstrukturen weniger stark beeinträchtigt als starke Säuren, sich deren Rückstände gut durch Sublimation entfernen lassen und eine vergleichsweise gute Zellverträglichkeit aufweisen. Je höher die Konzentration an zu lösenden Kollagenen, desto höhere Molaritäten von Essigsäure werden eingesetzt. In einer besonders bevorzugten Ausführungsform der sublimierbare Stoff daher 0,25 - 4 M Essigsäure, bevorzugt 0,5 - 3 M Essigsäure, ferner bevorzugt 0,5 M Essigsäure.

In einer bevorzugten Ausführungsform enthält die erste, zweite und/oder dritte Substanz 0,5 - 60 Gew.-% Polymer, bevorzugt 0,8 - 20 Gew.-% Polymer, ferner bevorzugt 1 - 15 Gew.-% Polymer. Höhere Polymeranteile führen zu einer stabileren Struktur der Trägermatrix.

In einer bevorzugten Ausführungsform enthält die Trockenmasse der ersten, zweiten und/oder dritten Substanz, unabhängig voneinander, 5 % - 65 % Glycosaminoglycane, wie beispielsweise Chondroitinsulfat. In einer ferner bevorzugten Ausführungsform enthält die Trockenmasse der ersten Substanz (NP-Vorstufe) 10 % - 65 % an Glycosaminoglycanen, die Trockenmasse der zweiten Substanz (iAF-Vorstufe) 10 % - 55 % an Glycosaminoglycanen und die Trockenmasse der dritten Substanz (oAF-Vorstufe) 5 % - 30 % an Glycosaminoglycanen.

In einer bevorzugten Ausführungsform zu Herstellung von Bandscheiben-Trägermatrices beträgt der Temperaturgradient zwischen 0,25 K/mm und 10 K/mm.

In einer bevorzugten Ausführungsform beträgt die interpolierte Erstarrungsgeschwindigkeit von 0,1 x10⁻² mm/s bis 10 x10⁻² mm/s.

In einer bevorzugten Ausführungsform ist die erste Schicht gebildet durch nasschemische Quervernetzung. Dies erfolgt beispielsweise durch Zugabe einer Carbondiimid-Lösung. Die Carbondiimid-Lösung liegt in Form eines Ethanol-WasserGemisches, welches 5 - 40 mM N-Hydroxysuccinimid, 50 - 250 mM 1-Ethyl-3-(3-dimethylaminopropyl)carbodiimid und 100 - 350 mM 2-Morpholinoethansulfonsäure enthält, vor. Die vorverfestigte NP-Vorstufe wird anschließend innerhalb eines Behälters, der einen weiteren formenden, isolierenden Körper enthält, platziert, und der freie Raum zwischen der vorverfestigten NP-Vorstufe und dem isolierenden Körper wird mit der zweiten Substanz (iAF-Vorstufe) gefüllt.

In einer alternativen Ausführungsform ist die erste Schicht gebildet indem die erste Substanz ungerichtet erstarrt wurde, beispielsweise bei einer einheitlichen Temperatur oder einem sehr geringen Temperaturgradienten von < 0,5 K/mm. Dazu wird die Substanz zur Herstellung der NP Zone (NP-Vorstufe) beispielsweise innerhalb eines formgebenden isolierenden Körpers, welcher sich wiederum innerhalb eines Behälters befinden kann ungerichtet erstarrt. Nach dem Abschluss der ungerichteten Erstarrung kann der isolierende Körper aus dem Behältnis entfernt und durch einen weiteren formgebenden isolierenden Körper ersetzt werden.

In den dadurch entstandenen Raum wird die iAF-Vorstufe eingefüllt und die Vorstufen werden erstarrt werden. Dabei wird die vorverfestigte NP Vorstufe ungerichtet erstarrt, während die iAF-Vorstufe gerichtet erstarrt wird. Nach dem Abschluss der gerichteten Erstarrung kann der isolierende Körper aus dem Behältnis entfernt werden. Nach der Erstarrung der zweiten Substanz (iAF-Vorstufe) wird der isolierende Körper entfernt und die dritte Substanz (oAF-Vorstufe) in dem freien Raum angeordnet und gerichtet erstarrt.

In einer bevorzugten Ausführungsform erfolgt das Erstarren der Vorstufen innerhalb eines mikrostrukturierten Behälters. Die Bildung von Kristallisationskeimen der sublimierbaren Zusätze entsteht bevorzugt an Stellen am Behälterboden, die durch die Mikrostrukturierung vorgegebenen sind. Durch eine entsprechende Mikrostrukturierung wird die räumliche Orientierung der Erstarrungsformen der sublimierbaren Zusätze gesteuert. Dies führt beispielsweise zu Lamellenstrukturen der gerichtet erstarrten Vorstufen (iAF und oAF), welche die ungerichtet erstarrte Vorstufe (NP) konzentrisch umschließen.

Die Trägermatrix ist zur Behandlung von Bandscheiben- Defekten, beispielsweise in Form von matrixgekoppelter Zelltransplantation, geeignet. Außerdem kann die erfindungsgemäße Trägermatrix zur Zellkultivierung eingesetzt werden.

### Beispiele

### 1. Aufbau der Erstarrungsapparatur

Die Erstarrungsapparatur ist wie in Figur 1 dargestellt aufgebaut. An Wärmetauscher gekoppelte Peltierelemente erzeugen einen Temperaturgradienten, der durch die Regelung des Stromflusses in den Peltierelementen gesteuert wird. In den Probenraum werden sukzessive Vorstufen (z.B. Subchondral (SC)-Suspension, untere Chondralzonen (CD)-Suspension und mittlere Chondralzonen (CM)-Suspension) eingeleitet und so innerhalb des Temperaturgradienten platziert. Durch den Temperaturgradienten kommt es innerhalb der Vorstufen zu einem unidirektionalen Eiskristallwachstum. Die Apparatur kann ferner temperierbare Körper, sowie ein die Vorstufen beinhaltendes Behältnis enthalten.

Der Probenraum (Figur 1 b) befindet sich in Zentrum der Isolationseinheit I1 und steht in thermischem Kontakt mit den temperierbaren Körpern W1 und W2, die sowohl oberhalb als auch unterhalb des Probenraums angebracht sind. Die temperierbaren Körpern W1 und W2 befinden sich innerhalb der Isolationseinheit I2 und sind an die Peltierelemente P1 und P2 gekoppelt, welche durch die Isolationseinheiten I3 und I4 fixiert werden. Diese innere Baugruppe befindet sich im Zentrum des Wärmetauscherrings A2, welcher zusammen mit den Wärmetauschereinheiten A1 und A3 eine äußere Baugruppe bilden.

### 2. Osteochondrale Trägermatrix zur Behandlung von Gelenkknorpeldefekten

### 2.1 Herstellung der Vorstufen

Die Vorstufen der einzelnen Schichten, welche die mittlere Chondralzone, die untere Chondralzone und die Subchondralzone nachbilden, wiesen folgende Zusammensetzungen aus:
mittlere Chondralzone (CM):
   1,0 Gew.-% Kollagen Typ II
   0,16 Gew.-% Chondroitinsulfat, und
   0,5 M Essigsäure als sublimierbarer Stoff;
untere Chondralzone (CD):
   1,0 Gew.-% Kollagen Typ II,
   0,2 Gew.-% Chondroitinsulfat, und
   0,5 M Essigsäure als sublimierbarer Stoff;
Subchondralzone (SC):
   0,8 Gew.-% Kollagen Typ I,
   0,8 Gew.-% resorbierbare Calciumphosphatphase Bruschit, und
   0,5 M Essigsäure als sublimierbarer Stoff.

Zur Herstellung der einzelnen Vorstufen wurde lyophilisiertes Kollagen Typ II bzw. Kollagen Typ I verwendet. Die Bestandteile der Vorstufen wurden bei Raumtemperatur für 30 min in Essigsäure verrührt und anschließend für 24 h bei 5° C quellen gelassen. Vor Gebrauch wurden die Vorstufen auf 15 °C vortemperiert.

### 2.2 Gefrierstrukturierung der Vorstufen

Die Gefrierstrukturierung erfolgte mit einer Erstarrungsapparatur, wie in Beispiel 1 beschrieben. In die innere Baueinheit der Erstarrungsapparatur wurde eine Polystyrol-Zellkulturschale als Behältnis, zur Aufnahme der Vorstufen eingesetzt. Durch eine elektrische Regelung der Peltierelemente wurde ein äußerer Temperaturgradient von 8 K/mm mit T_{Pelier1} -40 °C (unteres Peltierelement) und T_{Pelier2} 24 °C (oberes Peltierelement) eingestellt. Sobald sich die innere Baueinheit und das Behältnis nahe dem thermischen Gleichgewicht befanden, wurden 2 ml der Vorstufe für die Subchondralzone in das Behältnis eingespritzt und für 20 min erstarrt. Anschließend wurden 2 ml der Vorstufe der unteren Chondralzone in das Behältnis eingespritzt, so dass die untere Chondralzone unmittelbar auf der Subchondralzone gebildet wurde. Nach weiteren 20 min wurden 2 ml der Vorstufe der oberen Chondralzone in das Behältnis, d.h. unmittelbar auf die untere Chondralzone, eingespritzt. Die Vorstufen wurden für weitere 20 min erstarrt.

Anschließend wurde das Behältnis mit den erstarrten Vorstufen aus der Erstarrungsapparatur entfernt und gegebenenfalls bei -20 °C zwischengelagert.

### 2.3 Lyophilisation der gerichtet erstarrten Vorstufen

Das Behältnis mit den erstarrten Vorstufen wurde in das Arbeitsvolumen eines laufenden Lyophilisators eingebracht. Bei einem Druck von 0,08 mbar und einer Temperatur von -60 °C wurden die erstarrten Vorstufen 24 h lang lyophilisiert. Dabei wurden die erstarrten sublimierbaren Bestandteile der Vorstufen aus diesen sublimiert und entfernt.

### 2.4 Verfestigung des Werkstoffs

Die erstarrten Vorstufen wurden durch ein Dehydrothermalverfahren bei einem Druck von 0,08 mbar und einer Temperatur von 105°C für 210 min vorvernetzt. Anschließend wurde der Werkstoff durch nasschemische Quervernetzung weiter verfestigt. Dazu wurden die erstarrten und vorvernetzten Vorstufen in einem Druckbehälter platziert und, nach Erreichen des Arbeitsdrucks von 100 mbar, wurden 100 µl/mg Werkstoff einer Carbondiimid-Lösung hinzu gegeben. Als Carbondiimid-Lösung wurde ein 2:3 Ethanol-Wasser-Gemisch, welches 21 mM N-Hydroxysuccinimid, 52 mM 1-Ethyl-3-(3-dimethylaminopropyl)carbodiimid und 200 mM 2-Morpholinoethansulfonsäure enthielt verwendet. 45 Sekunden nach der Infiltration der Werkstoffporen wurde der Druckbehälter belüftet. Nach 24 h Reaktionszeit wurde die Trägermatrix dreimal in destilliertem Wasser gewaschen.

### 2.5 Aufbringung von funktionalisierter Polymerfasern

Als reibungsmindernde Oberfläche (CS) wurden funktionalisierte Polymerfasern auf die mittlere chondrale Zone der Trägermatrix aufgebracht. Diese wurden mittles elektrostatischem Verspinnen, nach dessen Verfestigung, auf den Werkstoff aufgebracht. Die experimentelle Vorgehensweise erfolgte wie in Grafahrend et al. (2010) beschrieben.

### 2.6 Eigenschaften der Trägermatrix

Die fertige vierschichtige Trägermatrix bildet den nativen Knorpel nach, und umfasst eine Subchondralzone, eine untere Chondralzone, eine mittlere Chondralzone und eine reibungsmindernde Oberfläche (Figuren 2, 3 und 8). Lichtmikroskopische und elektronenmikroskopische Aufnahmen der Trägermatrix zeigen deren langgestreckte anisotrope Poren sowohl im Alginatmodel (Beispiel 6, Figur 3) als auch in der Kollagenträgermatrix (Figur 4). Diese Strukturen sind durch die gerichteten Kristalle der Essigsäure, die beim Erstarren der Vorstufen gebildet wurden, und deren anschließendes Sublimieren, entstanden. Zu dieser Porenstruktur senkrecht angeordnet verläuft die Faserstruktur der reibungsmindernden Oberfläche. (Figur 8)

### 3. Osteochondrale Trägermatrix zur Behandlung von Gelenkknorpeldefekten

### 3.1 Herstellung der Vorstufen

Die Vorstufe der einzelnen Schichten, welche die mittlere Chondralzone, die untere Chondralzone und die Subchondralzone nachbilden, wiesen folgende Zusammensetzungen auf:
mittlere Chondralzone (CM):
   2,5 Gew.-% Kollagen Typ II
   0,5 Gew.-% Chondroitinsulfat, und
   0,5 M Essigsäure als sublimierbarer Stoff;
untere Chondralzone (CD):
   2,5 Gew.-% Kollagen Typ II,
   0,8 Gew.-% Chondroitinsulfat, und
   0,5 M Essigsäure als sublimierbarer Stoff;
Subchondralzone (SC):
   1,0 Gew.-% Kollagen Typ I,
   0,5 Gew.-% resorbierbare Calciumphosphatphase Bruschit, und
   0,5 M Essigsäure als sublimierbarer Stoff.

Die einzelnen Vorstufen wurden wie in Beispiel 2 hergestellt.

### 3.2 Gefrierstrukturierung der Vorstufen

Die Gefrierstrukturierung erfolgte wie in Beispiel 2 dargestellt bei einem äußeren Temperaturgradienten von 6,25 K/mm mit T_{Pelier1} -32 °C (unteres Peltierelement) und T_{Pelier2} 7,5 °C (oberes Peltierelement) und einer daraus folgenden interpolierten Erstarrungsgeschwindigkeit von 0,27 x 10⁻² mm/s.

### 3.3 Lyophilisation der gerichtet erstarrten Vorstufen

Die Lyophilisation erfolgte wie in Beispiel 2 dargestellt bei einem Druck von 0,08 mbar und einer Temperatur von -60°C für 17 h.

### 3.4 Verfestigung des Werkstoffs

Die Verfestigung des Werkstoffs erfolgte wie in Beispiel 2 dargestellt.

### 4. Trägermatrix zur Behandlung von Meniskusdefekten

### 4.1 Herstellung der Vorstufen

Die Vorstufe der einzelnen Schichten, welche den äußeren (OM) und den inneren Meniskusbereich (IM) nachbilden (Figur 5 a), wiesen folgende Zusammensetzungen auf:
äußerer Meniskusbereich (OM):
   1,5 Gew.-% Kollagen Typ I
   0,012 Gew.-% Chondroitinsulfat, und
   0,5 M Essigsäure als sublimierbarer Stoff;
innerer Meniskusbereich (IM):
   1,8 Gew.-% Kollagen Typ II
   12 Gew.-% Kollagen Typ I
   0,06 Gew.-% Chondroitinsulfat, und
   0,5 M Essigsäure als sublimierbarer Stoff.

Die einzelnen Vorstufen wurden wie in Beispiel 2 hergestellt.

### 4.2 Gefrierstrukturierung der Vorstufen

Die Gefrierstrukturierung erfolgte mit einer Erstarrungsapparatur, wie in Beispiel 1 beschrieben. In die innere Baueinheit der Erstarrungsapparatur wurde ein Behältnis eingesetzt, das die negative Form eines Meniskus darstellte (Figur 5 b) und ein als Platzhalter für den inneren Meniskusbereich fungierenden isolierenden Körper enthielt. In dieses Behältnis wurde zunächst die Vorstufe des äußeren Meniskusbereichs eingebracht und erstarrt. Anschließend wurde der isolierende Körper entfernt und die Vorstufe des inneren Meniskusbereichs eingebracht und erstarrt. Durch die Form des Behältnisses erhielt die entstandene Trägerstruktur die Form eines Meniskus (Figur 5 c).

Durch eine elektrische Regelung der Peltierelemente wurde ein äußerer Temperaturgradient von 4,5 K/mm mit T_{Peltier1} = -20 °C (unteres Peltierelement) und T_{Peltier2} = 16 °C (oberes Peltierelement) eingestellt. Ein isolierender Körper, der als Platzhalter diente und die Form des inneren Meniskusbereichs aufwies, wurde in das Behältnis gesetzt Sobald sich die innere Baueinheit und das Behältnis mit isolierendem Körper nahe dem thermischen Gleichgewicht befanden, wurden 4 ml der Vorstufe für den äußeren Meniskusbereich in das Behältnis eingespritzt und für 25 min erstarrt.. Dadurch wurde die innere Kante des äußeren Meniskusbereichs geformt. Nach dem Erstarren der Vorstufe des äußeren Meniskusbereichs wurde der Platzhalter aus dem Behältnis entfernt und 2 ml der Vorstufe für den inneren Meniskusbereich in das Behältnis eingespritzt, so dass der inneren Meniskusbereich unmittelbar auf dem äußeren Meniskusbereich gebildet wurde. Die Vorstufen wurden für weitere 15 min erstarrt.

Anschließend wurde das Behältnis mit den erstarrten Vorstufen aus der Erstarrungsapparatur entfernt und gegebenenfalls bei -20 °C bis zur Weiterverarbeitung zwischengelagert.

### 4.3 Lyophilisation der gerichtet erstarrten Vorstufen

Die Lyophilisation erfolgte wie in Beispiel 2 dargestellt bei einem Druck von 0,08 mbar und einer Temperatur von -60°C für 24 h.

### 4.4 Verfestigung des Werkstoffs

Die Verfestigung des Werkstoffs erfolgte wie in Beispiel 2 dargestellt.

### 4.5 Eigenschaften der Trägermatrix

Die fertige zweischichtige Trägermatrix entspricht der äußeren Form (Figur 5 c) und der inneren Struktur eines nativen Meniskus (Figur 6 a, b).. Die Trägermatrix ist von lamellaren Poren durchzogen (Figur 6 a, b). Beim Erstarren der Vorstufen der Meniskusbereiche kommt es zum Überwachsen der zentralen Kristalle durch benachbarte Kristalle, wodurch lamellenförmige Poren entsprechend der nativen Gewebestruktur überwiegend horizontal durch die Meniskus-Trägermatrix verlaufen.

### 5. Trägermatrix zur Behandlung von Bandscheibendefekten

### 5.1 Herstellung der Vorstufen

Die Vorstufe der einzelnen Schichten, welche den Nucleus pulposus (NP), den inneren Anulus fibrosus (iAF) und den äußeren Anulus fibrosus (oAF) nachbilden, wiesen folgende Zusammensetzungen auf:
Nucleus pulposus (NP):
   4 Gew.-% Kollagen Typ II
   2 Gew.-% Chondroitinsulfat, und
   0.5 M Essigsäure als sublimierbarer Stoff;
innerer Anulus fibrosus (iAF):
   2 Gew.-% Kollagen Typ II,
   0,8 Gew.-% Chondroitinsulfat, und
   0,5 M Essigsäure als sublimierbarer Stoff;
äußerer Anulus fibrosus (oAF):
   1 Gew.-% Kollagen Typ I,
   0,15 Gew.-% Chondroitinsulfat, und
   0,5 M Essigsäure als sublimierbarer Stoff.

Die einzelnen Vorstufen wurden wie in Beispiel 2 hergestellt.

### 5.2 Gefrierstrukturierung der Vorstufen

Die Gefrierstrukturierung erfolgt mit einer Erstarrungsapparatur, wie in Beispiel 1 beschrieben. In die innere Baueinheit der Erstarrungsapparatur wird ein Behältnis angeordnet, das nach seiner Form einer Bandscheibe entspricht. Am Boden dieses Behältnisses befindet sich eine Mikrostruktur, bestehend aus linearen Vertiefungen. Diese breiten sich, ausgehend von dem Bereich des Nucleus pulposus (NP), in radialer Richtung aus. Die Mikrostruktur dient als Kristallisationspunkt für die Erstarrung der Vorstufen des inneren Anulus fibrosus (iAF) und des äußeren Anulus fibrosus (oAF).

Das mikrostrukturiertes Behältnis wurde in die innere Baueinheit der Erstarrungsapparatur eingelegt, wobei isolierende Körper als Platzhalter für die iAF und oAF fungierten. Die Platzhalter nahmen ca. 36 % des zur Trägermatixherstellung zu Verfügung stehenden Volumens ein. Durch elektrische Regelung der Peltierelemente wurde zuerst ein äußerer Temperaturgradient von 0,25 K/mm eingestellt (T_{Peltier1} = -22 °C; T_{Peltier2} = -20 °C). Sobald sich die innere Baueinheit mitsamt der darin befindlichen formgebenden Körper nahe dem thermischen Gleichgewicht befand, wurden 0,7 ml der Vorstufe des NP im Zentrum des Behältnisses innerhalb der Probenkammer eingefüllt und 15 min erstarrt. Anschließend wurde durch elektrische Regelung der Peltierelemente ein äußerer Temperaturgradient von 8,5 K/mm eingestellt werden (T_{Peltier1} = -40 °C; T_{Peltier2} = 28 °C). Sobald sich die innere Baueinheit mitsamt der darin befindlichen formgebenden Körper nahe dem thermischen Gleichgewicht befand, wurde der Platzhalter für die iAF Vorstufe entfernt und 1,2 ml der Vorstufe des iAF zwischen der dem NP entsprechenden Schicht und dem isolierender Platzhalter für die oAF Vorstufe eingefüllt. Der Platzhalter für die oAF Vorstufe befand sich am äußeren Rand des Behältnisses und füllte ca. 18 % des Volumens des Behältnisses aus. Nach weiteren 10 min Gefrierstrukturierung wurde der Platzhalter entfernt und an seiner Stelle 1,5 ml der Vorstufe des oAF zugeführt. Nach weiteren 10 min Gefrierstrukturierung wurde das Behältnis mit den gemeinsam gerichtet erstarrten Vorstufen entnommen und gegebenenfalls bei -20 °C bis zur Weiterverarbeitung zwischengelagert.

### 5.3 Lyophilisation der gerichtet erstarrten Vorstufen

Die Lyophilisation erfolgte wie in Beispiel 2 dargestellt bei einem Druck von 0,08 mbar und einer Temperatur von -60°C für 17 h.

### 5.4 Verfestigung des Werkstoffs

Die Verfestigung des Werkstoffs erfolgte wie in Beispiel 2 dargestellt.

### 5.5 Eigenschaften der Trägermatrix

Aufgrund des nur sehr geringen Temperaturgradienten bei der Erstarrung des dem NP entsprechende Bereichs der Trägermatrix kommt es zu einer ungerichteten Erstarrung. Dadurch weist dieser Bereich eine isotrope Porenstruktur auf. Die daran angrenzenden Bereiche des iAF und des oAF weisen auf Grund der gerichteten Erstarrung im Temperaturgradienten hingegen eine lamellare anisotrope Porenstruktur auf. Durch die Mikrostrukturierung am Boden des Behältnisses zeigen die anisotropen Poren ein konzentrisch um den Bereich des NP angeordnet Muster (Figur 7 b)

### 6. Alginatmodell für eine Trägermatrix zur Behandlung von Meniskusdefekten

### 6.1 Herstellung der Vorstufen

Die Vorstufen der einzelnen Schichten bestanden zu Modellzecken aus 5,5 % Alginat, gelöst in destilliertem Wasser.

### 6.2 Gefrierstrukturierung der Vorstufen

Die Gefrierstrukturierung erfolgte mit einer Erstarrungsapparatur, wie in Beispiel 1 beschrieben. In die innere Baueinheit der Erstarrungsapparatur wurde ein Behältnis eingesetzt, das die negative Form eines Meniskus darstellte (Figur 5 b). In dieses Behältnis wurde zunächst die Vorstufe des äußeren Meniskusbereichs eingebracht und erstarrt und anschließend die Vorstufe des inneren Meniskusbereichs eingebracht und erstarrt. Durch die Form des Behältnisses erhielt die entstandene Trägerstruktur die Form eines Meniskus (Figur 5 c).

Durch eine elektrische Regelung der Peltierelemente wurde ein äußerer Temperaturgradient von 1,8 K/mm mit T_{Peltier1} = -20 °C (unteres Peltierelement) und T_{Peltier2} = 5 °C (oberes Peltierelement) eingestellt. Sobald sich die innere Baueinheit und das Behältnis mit einem isolierenden Körper, der als Platzhalter diente und die Form des inneren Meniskusbereichs besaß, nahe dem thermischen Gleichgewicht befanden, wurden 8 ml der Vorstufe für den äußeren Meniskusbereich in das Behältnis eingespritzt und für 30 min erstarrt. Dadurch wurde die innere Kante des äußeren Meniskusbereichs geformt. Nach dem Erstarren der Vorstufe des äußeren Meniskusbereichs wurde der Platzhalter aus dem Behältnis entfernt und 4 ml der Vorstufe für den inneren Meniskusbereich in das Behältnis eingespritzt, so dass der innere Meniskusbereich unmittelbar auf dem äußeren Meniskusbereich gebildet wurde. Die Vorstufen wurden für weitere 15 min erstarrt.

Anschließend wurde das Behältnis mit den erstarrten Vorstufen aus der Erstarrungsapparatur entfernt und gegebenenfalls bei -20 °C bis zur Weiterverarbeitung zwischengelagert.

### 6.3 Lyophilisation der gerichtet erstarrten Vorstufen

Die Lyophilisation erfolgte wie in Beispiel 2 dargestellt bei einem Druck von 0,08 mbar und einer Temperatur von -60°C für 24 h.

### 6.4 Verfestigung des Werkstoffs

Die Verfestigung des Werkstoffs erfolgte durch nasschemische Quervernetzung. Dazu die wurde gefriergetrockneten Struktur in einem Druckbehälter platziert und, nach Erreichen des Arbeitsdrucks von 100 mbar, wurden 50 µl/mg Werkstoff einer 1M CaCl₂ Lösung hinzu gegeben. 45 Sekunden nach der Infiltration der Werkstoffporen wurde der Druckbehälter belüftet. Nach 24 h Reaktionszeit wurde die Trägermatrix dreimal in destilliertem Wasser gewaschen.

### 7. Alginatmodell für eine Trägermatrix zur Behandlung von Bandscheibendefekten

### 7.1 Herstellung der Vorstufen

Die Vorstufen der einzelnen Schichten bestanden zu Modellzecken aus 5,5 % Alginat, gelöst in destilliertem Wasser und waren zur visuellen Unterscheidung der einzelnen Schichten mit unterschiedlichen Farbstoffen versetzt. Vor Gebrauch wurden die Vorstufen auf 15 °C vortemperiert.

### 7.2 Gefrierstrukturierung der Vorstufen

Die Gefrierstrukturierung erfolgte wie in Beispiel 5 dargestellt.

### 7.3 Lyophilisation der gerichtet erstarrten Vorstufen

Die Lyophilisation erfolgte wie in Beispiel 2 dargestellt bei einem Druck von 0,08 mbar und einer Temperatur von -60°C für 24 h.

### 7.4 Verfestigung des Werkstoffs

Die Verfestigung des Werkstoffs erfolgte wie in Beispiel 6 dargestellt.

### Referenzen

A. Tampieri, M. Sandri, E. Landi, D. Pressato, S. Francioli, R. Quarto, et al., Biomaterials Design of graded biomimetic osteochondral composite scaffolds, Biomaterials. 29 (2008) 3539-3546.
T.J. Klein, S.C. Rizzi, J.C. Reichert, N. Georgi, J. Malda, W. Schuurman, et al., Strategies for Zonal Cartilage Repair using Hydrogels, Macromolecular Bioscience. 9 (2009) 1049-1058.
D. Grafahrend, K.-H. Heffels, M.V. Beer, P. Gasteier, M. Möller, G. Boehm, et al., Degradable polyester scaffolds with controlled surface chemistry combining minimal protein adsorption with specific bioactivation., Nature Materials. 10 (2010) 67-73.
DE 197 51 031 A1
EP 1 858 562 B1

## Patentansprüche

1. Verfahren zur Herstellung eines mehrschichtigen Werkstoffs mit anisotropen Poren, umfassend die Schritte
- Bereitstellen eines Temperaturgradienten durch zwei einander gegenüber liegend angeordnete temperierbare Körper,
- Anordnen und Erstarren einer ersten Substanz, die mindestens einen sublimierbaren Stoff enthält, in dem Temperaturgradienten, um eine erste Schicht zu bilden,
- Anordnen und Erstarren mindestens einer weiteren Substanz, die mindestens einen sublimierbaren Stoff enthält, in dem Temperaturgradienten, um mindestens eine weitere Schicht zu bilden, die an die vorhergehende Schicht anschließt,
- Sublimieren des Stoffs und
- Verfestigen der Schichten.

2. Verfahren nach Anspruch 1, **dadurch gekennzeichnet, dass** der Temperaturgradient zwischen 0,5 K/mm und 200 K/mm, bevorzugt zwischen 2,5 K/mm und 25 K/mm, ferner bevorzugt zwischen 5 K/mm und 15 K/mm beträgt.

3. Verfahren nach Anspruch 1 oder 2, **dadurch gekennzeichnet, dass** die erste und/oder eine weitere Substanz unabhängig voneinander mindestens ein Polymer oder dessen Monomere enthält.

4. Verfahren nach Anspruch 3, **dadurch gekennzeichnet, dass** das Polymer ausgewählt ist aus der Gruppe bestehend aus Peptiden, Proteinen, bevorzugt Strukturproteinen, Polysacchariden, und Mischungen davon.

5. Verfahren nach einem der vorhergehenden Ansprüche, **dadurch gekennzeichnet, dass** der sublimierbare Stoff ausgewählt ist aus der Gruppe bestehend aus wässrigen Lösungsmitteln, polaren Lösungsmitteln, unpolaren Lösungsmitteln, organischen Säuren, organischen Basen, mineralischen Säuren und mineralischen Basen.

6. Verfahren nach einem der vorhergehenden Ansprüche, **dadurch gekennzeichnet, dass** die erste Substanz und mindestens eine weitere Substanz den gleichen sublimierbaren Stoff aufweisen.

7. Verfahren nach Anspruch 6, **dadurch gekennzeichnet, dass** jede weitere Substanz den gleichen sublimierbaren Stoff aufweist.

8. Verfahren nach einem der vorhergehenden Ansprüche, **dadurch gekennzeichnet, dass** mindestens einer der temperierbaren Körper eine Mikrostrukturierung aufweist.

9. Verfahren nach Anspruch 1, ferner umfassend den Schritt
- Anordnen einer Schicht funktionalisierter Polymerfasern auf der Trägermatrix als äußerste Schicht.

10. Verfahren nach Anspruch 1, **dadurch gekennzeichnet, dass** das Verfestigen durch nasschemische Quervernetzung, Dehydrothermalverfahren, enzymatische Quervernetzung, nicht-enzymatische Glykation, UV-Bestrahlung, Gamma-Bestrahlung, Sinterung, Infiltration des Werkstoffs oder eine Kombination daraus erfolgt.

## Claims

1. Process for the production of a multi-layered material having anisotropic pores, comprising the steps of
- providing a temperature gradient by two temperature-controllable bodies arranged opposite one another,
- arranging and solidifying in the temperature gradient a first substance which contains at least one sublimable compound in order to form a first layer,
- arranging and solidifying in the temperature gradient at least one further substance which contains at least one sublimable compound in order to form at least one further layer adjacent to the preceding layer,
- subliming the compound and
- consolidating the layers.

2. Process of claim 1, **characterized in that** the temperature gradient is between 0.5 K/mm and 200 K/mm, preferably between 2.5 K/mm and 25 K/mm, further preferred between 5 K/mm and 15 K/mm.

3. Process of claim 1 or 2, **characterized in that** the first and/or a further substance, independently of each other, contains at least one polymer or monomers thereof.

4. Process of claim 3, **characterized in that** the polymer is selected from the group consisting of peptides, proteins, preferably structural proteins, polysaccharides, and mixtures thereof.

5. Process of any one of the preceding claims, **characterized in that** the sublimable compound is selected from the group consisting of aqueous solvents, polar solvents, non-polar solvents, organic acids, organic bases, mineral acids and mineral bases.

6. Process of any one of the preceding claims, **characterized in that** the first substance and at least one further substance have the same sublimable compound.

7. Process of claim 6, **characterized in that** every further substance has the same sublimable compound.

8. Process of any one of the preceding claims, **characterized in that** at least one of the temperature-controllable bodies has a microstructuring.

9. Process of claim 1, further comprising the step of
- arranging a layer of functionalized polymer fibres on the support matrix as outermost layer.

10. Process of claim 1, **characterized in that** the consolidating is carried out by wet chemical crosslinking, dehydrothermal processes, enzymatic crosslinking, non-enzymatic glycation, UV irradiation, gamma irradiation, sintering, infiltration of the material or a combination thereof.

## Revendications

1. Procédé pour la fabrication d'un matériau multicouche avec des pores anisotropes, comprenant les étapes consistant à
- mettre en place un gradient de température entre deux corps susceptible d'être tempéré agencés de manière à se trouver opposés l'un à l'autre,
- agencer et solidifier une première substance, qui contient au moins une matière sublimable, dans le gradient de température, pour former une première couche,
- agencer et solidifier au moins une autre substance, qui contient au moins une matière sublimable, dans le gradient de température, pour former au moins une autre couche, qui se raccorde à la couche précédente,
- sublimer la matière et
- consolider les couches.

2. Procédé selon la revendication 1, **caractérisé en ce que** le gradient de température se trouve entre 0,5 K/mm et 200 K/mm, et de préférence entre 2,5 K/mm et 25 K/mm, et plus préférentiellement entre 5 K/mm et 15 K/mm.

3. Procédé selon la revendication 1 ou 2, **caractérisé en ce que** la première et/ou une autre substance contient indépendamment l'une de l'autre au moins un polymère ou les monomères de celle-ci.

4. Procédé selon la revendication 3, **caractérisé en ce que** le polymère est sélectionné dans le groupe consistant en des peptides, des protéines, de préférence des protéines structurales, des polysaccharides et de mélanges de ces derniers.

5. Procédé selon l'une quelconque des revendications précédentes, **caractérisé en ce que** la matière sublimable est sélectionnée dans le groupe consistant en des solvants aqueux, des solvants polaires, des solvants non polaires, des acides organiques, des bases organiques, des acides minéraux et des bases minérales.

6. Procédé selon l'une quelconque des revendications précédentes, **caractérisé en ce que** la première substance et au moins une autre substance présentent la même matière sublimable.

7. Procédé selon la revendication 6, **caractérisé en ce que** chaque autre substance présente la même matière sublimable.

8. Procédé selon l'une quelconque des revendications précédentes, **caractérisé en ce qu'**au moins l'un des corps susceptible d'être tempéré présente une microstructure.

9. Procédé selon la revendication 1, comprenant en outre l'étape consistant à
- agencer une couche de fibres polymères fonctionnalisées sur la matrice de support en tant que couche la plus extérieure.

10. Procédé selon la revendication 1, **caractérisé en ce que** la consolidation se fait par réticulation transversale humide, procédé de déshydratation thermique, réticulation transversale enzymatique, glycation non enzymatique, irradiation par les rayons ultraviolet, irradiation par les rayons gamma, frittage, infiltration du matériau ou une combinaison de ces techniques.
